# EUROPÄISCHE PATENTSCHRIFT

(11) **EP 3 274 329 B1**
(45) Veröffentlichungstag und Bekanntmachung des Hinweises auf die Patenterteilung: **16.09.2020**
(21) Anmeldenummer: 16714323.9
(22) Anmeldetag: 23.03.2016
(51) Int. Cl.: C07C 215/50, C08G 59/50, C09D 163/00, C08L 63/00

(54) **KALTHÄRTENDER EPOXIDHARZ-PRIMER ODER -KLEBSTOFF**
COLD CURING EPOXY RESIN PRIMER OR ADHESIVE
PRIMAIRE OU ADHÉSIF ÉPOXYDIQUE DURCISSANT À FROID

(30) Priorität: 23.03.2015 EP 15160411
(43) Veröffentlichungstag der Anmeldung: 31.01.2018
(73) Patentinhaber: Sika Technology AG, 6340 Baar (CH)
(72) Erfinder: KASEMI, Edis, 8046 Zürich (CH); KRAMER, Andreas, 8006 Zürich (CH); STADELMANN, Ursula, 8046 Zürich (CH); BURCKHARDT, Urs, 8049 Zürich (CH)
(74) Vertreter: Sika Patent Attorneys
(86) Internationale Anmeldenummer: PCT/EP2016/056362
(87) Internationale Veröffentlichungsnummer: WO 2016/151007

(56) Entgegenhaltungen:
- EP-A1- 2 546 276
- US-A- 3 217 039
- US-A- 4 399 268
- DATABASE CA [Online] CHEMICAL ABSTRACTS SERVICE, COLUMBUS, OHIO, US; HARADA, YASUHIRO ET AL: "Polyolefin-lined steel article with high interlayer adhesion and corrosion resistance", XP002757975, gefunden im STN Database accession no. 2004:898774 -& JP 2004 299238 A (JFE STEEL KK) 28. Oktober 2004 (2004-10-28)
- DATABASE CA [Online] CHEMICAL ABSTRACTS SERVICE, COLUMBUS, OHIO, US; ZHANG, WENQUAN ET AL: "Method for preparing exfoliation-type montmorillonite/epoxy resin composite", XP002757976, gefunden im STN Database accession no. 2011:637068 -& CN 102 061 061 A (WUXI ARCO CHEMICAL CO LTD) 18. Mai 2011 (2011-05-18)
- DATABASE REGISTRY [Online] CHEMICAL ABSTRACTS SERVICE, COLUMBUS, OHIO, US; 8. Oktober 1999 (1999-10-08), XP002757977, Database accession no. 244020-66-4
- DATABASE REGISTRY [Online] CHEMICAL ABSTRACTS SERVICE, COLUMBUS, OHIO, US; 7. November 2008 (2008-11-07), XP002757978, Database accession no. 1071587-03-5

## Beschreibung

### Technisches Gebiet

Die Erfindung betrifft das Gebiet der Härter für Epoxidharze, Epoxidharz-Zusammensetzungen und deren Anwendung als Primer oder Klebstoff.

### Stand der Technik

Auf Epoxidharzen basierende Primer werden oft als Voranstrich bzw. Grundierung in Beschichtungen mit Mehrschichtaufbauten eingesetzt, vor allem im Aussenbereich, beispielsweise in Bodenbelägen, Parkhausbeschichtungen, Brückenabdichtungen, Balkonbeschichtungen oder Korrosionsschutz-Beschichtungen. Dabei soll der Primer eine niedrige Viskosität aufweisen, damit er gut verfliesst, den Untergrund bzw. das Substrat gut benetzt und bis zu einem gewissen Grad in dieses eindringt, um Poren und Risse zu verschliessen und das Substrat damit zu verfestigen. Weiterhin soll der Primer bei Umgebungstemperatur eine genügend lange Topfzeit aufweisen, damit er einfach von Hand verarbeitet werden kann, und dann schnell aushärten, auch in der Kälte, damit die mit dem Primer grundierte Fläche schon bald weiter bearbeitet werden kann. Nach der Aushärtung soll der Primer einen festen, nichtklebrigen Film bilden, der eine gute Haftung zum Substrat und beim Überdecken eine gute Haftung zur Deckschicht aufbaut und so als Haftbrücke zwischen Substrat und Deckschicht dient. Im Fall von porösen mineralischen Substraten, insbesondere zementösen Substraten wie Beton oder Mörtel, dient der Primer insbesondere zum Verschliessen der Poren und somit als Sperre gegen Feuchtigkeit von unten, sowie zum Binden von Staub.
Um diese Eigenschaften zu erreichen, werden als Härter für Primer im Stand der Technik oft sogenannte Mannich-Basen eingesetzt, welche Umsetzungsprodukte von Phenolen, Formaldehyd und Aminen darstellen. Mannich-Basen ermöglichen die geforderten hohen Aushärtungsgeschwindigkeiten, insbesondere auch bei kühlen Umgebungstemperaturen im Bereich von 5 bis 15 °C, und sind nicht anfällig auf die als "Blushing" bekannten Aushärtungsstörungen verursacht durch Salzbildung mit CO₂ aus der Luft. Aufgrund ihrer Herstellung enthalten sie einen beträchtlichen Anteil an freien toxischen Phenolen, wie beispielsweise Phenol, tert.Butylphenol oder Nonylphenol. Solche Phenole sind für Mensch und Umwelt giftig (Reproduktionstoxizität, Gewässertoxizität) und erfordern deshalb Vorsichts- bzw. Schutzmassnahmen bei der Verarbeitung, Lagerung und beim Transport. Bei der Aushärtung werden sie nicht chemisch ins Polymer eingebunden, weshalb sie durch Ausgasen oder Auswaschen in die Umwelt entweichen und somit auch langfristig eine Gefahr darstellen können. Bisher bekannte Phenol-freie Ersatzlösungen für Härter auf Basis von Mannich-Basen weisen allesamt Schwächen in der technischen Leistungsfähigkeit auf und sind entweder zu hochviskos, was eine starke Verdünnung mit unerwünschten VOC-Lösemitteln erfordert, zu empfindlich auf Blushing, härten zu langsam aus, insbesondere in der Kälte, oder führen nach der Aushärtung zu weichen, mechanisch wenig belastbaren Polymeren.
US 4,399,268 beschreibt phenolgruppen-haltige Umsetzungsprodukte aus der einfachen Alkylierung von m-Xylylendiamin, darunter Reaktionsprodukte aus der reduktiven Alkylierung mit Hydroxyaldehyden, welche geeignet sind als Härter für Beschichtungen mit hoher Chemikalienbeständigkeit, verwendbar in Beschichtungen mit Lebensmittelkontakt, zum Beispiel in Konservendosen. Beim beschriebenen Verfahren werden hochviskose Reaktionsprodukte und Zusammensetzungen erhalten. Eine Verwendung als Primer ist nicht beschrieben.
US 8,729,213 beschreibt benzylierte Polyamin-Härter und deren Verwendung in Epoxidharzen. Beschrieben sind hauptsächlich Reaktionsprodukte aus der reduktiven Alkylierung von Metaxylylendiamin mit Benzaldehyd in verschiedenen Verhältnissen. Solche Reaktionsprodukte sind zwar sehr niedrigviskos, weisen aber in der Verwendung als Härter für Primer in der Kälte eine ungenügende Reaktionsgeschwindigkeit auf. Erwähnt ist auch die Möglichkeit, Reaktionsprodukte ausgehend von Vanillin (4-Hydroxy-2-methoxybenzaldehyd) anstelle von Benzaldehyd einzusetzen. Solche Reaktionsprodukte sind aber sehr hochviskos und für Primer deshalb wenig vorteilhaft.
EP2546276 beschreibt Härter für Epoxidharze, welche über eine reduktive Alkylierung eines primären Amins mit einer Carbonylverbindung hergestellt werden, so zum Beispiel auch disalicyliertes MXDA. Die Härter aus EP2546276 sind jedoch bezüglich ihrer Verdünnungswirkung auf das Epoxidharz verbesserungswürdig.

### Darstellung der Erfindung

Aufgabe der Erfindung ist es, eine Epoxidharz-Zusammensetzung für die Verwendung als Primer zur Verfügung zu stellen, welche bei Umgebungstemperatur, insbesondere auch in der Kälte, schnell aushärtet, genügend niedrigviskos für die Anwendung auf porösen mineralischen Substraten ist und weitgehend frei von toxischen Phenol-Verbindungen wie Phenol, tert.Butylphenol oder Nonylphenol ist.
Überraschenderweise wird diese Aufgabe mit einer Epoxidharz-Zusammensetzung wie in Anspruch 1 beschrieben gelöst. Das in der Zusammensetzung enthaltene Reaktionsprodukt ist in einem einfachen Prozess aus gut erhältlichen Ausgangsstoffen herstellbar und enthält keine freien Phenole wie Phenol, tert.-Butylphenol oder Nonylphenol. Aufgrund der starken Verdünnungswirkung des Reaktionsprodukts ist die Zusammensetzung überraschend niedrigviskos, auch ohne niedrigmolekulare Bestandteile wie freie Phenole oder Lösemittel. Die Zusammensetzung weist eine ähnlich schnelle Aushärtung auf wie solche auf der Basis von konventionellen Mannich-Basen, insbesondere auch bei kalten Umgebungstemperaturen unterhalb von 20 °C oder unterhalb von 10 °C. Die schnelle Aushärtung ist überraschend, da die phenolische Gruppe des Reaktionsprodukts bei der Aushärtung chemisch ins entstehende Epoxid-Polymer eingebunden wird und man annehmen würde, dass ihre beschleunigende Wirkung dadurch abnimmt. Die erreichbare Zeit bis zur Klebefreiheit ist deutlich kürzer und die Endfestigkeit deutlich höher als beim Einsatz von vergleichbaren Reaktionsprodukten, welche ausgehend von ähnlichen Aminen hergestellt wurden.
Die niedrige Viskosität und gute Verdünnungswirkung des Reaktionsprodukts ist ebenfalls überraschend. Entsprechende Reaktionsprodukte abgeleitet von zu den Carbonylverbindungen der Formel (II) analogen Carbonylverbindungen, bei welchen die phenolische OH-Gruppe in meta- oder para-Stellung zum Carbonyl-Substituenten steht, sind um ein Vielfaches höherviskos.
Die Epoxidharz-Zusammensetzung nach Anspruch 1 ist somit hervorragend geeignet als Primer, insbesondere für die Verwendung auf porösen mineralischen Substraten sowie bei kalten Umgebungsbedingungen, wie sie auf Baustellen typisch sind. Die niedrige Viskosität ermöglicht dabei eine gute Benetzung und Eindringtiefe in das poröse Substrat, wobei die Poren gefüllt und verschlossen werden. Die schnelle Aushärtung ermöglicht, dass der Primer bzw. die Grundierung schon bald nach der Applikation begehbar ist und/oder überdeckt werden kann.
Weiterhin eignet sich die Epoxidharz-Zusammensetzung als Klebstoff, insbesondere für die Verwendung bei kalten Temperaturen, beispielsweise im Freien auf Baustellen oder in ungeheizten Industriehallen. Aufgrund der schnellen Aushärtung ist eine Verklebung auch bei kalten Temperaturen schon nach kurzer Zeit belastbar, was einen raschen Baufortschritt und kurze Taktzeiten erlaubt. Die niedrige Viskosität ermöglicht einen hohen Füllgrad des Klebstoffes mit beispielsweise Quarzmehl oder Quarzsand, was beispielsweise für einen Einsatz als Klebemörtel vorteilhaft ist.
Die Endfestigkeit und Beständigkeit der Zusammensetzung ist sehr hoch und befriedigt höchste Ansprüche. Zudem ist die Zusammensetzung fast farblos und zeigt eine überraschend geringe Neigung zum Vergilben unter Lichteinfluss, trotz des hohen aromatischen Anteils im darin enthaltenen Reaktionsprodukt. Dies ermöglicht ein hohes Mass an Gestaltungsspielraum beim Einsatz als Primer oder Klebstoff, indem beispielsweise eine sichtbare Klebefuge nicht übermalt werden muss, ein Primer in einem Beschichtungsaufbau dünnschichtig oder mit schwacher Pigmentierung überdeckt werden kann oder im Randbereich von Klebefugen oder Beschichtungen ganz ohne Überdeckung bleiben kann, ohne dass dies zu sichtbarer Farbveränderung und ästhetischen Einbussen führt.
Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist die Verwendung einer Epoxidharz-Zusammensetzung enthaltend mindestens ein Reaktionsprodukt aus der Umsetzung von mindestens einem Amin der Formel (I) mit mindestens einer Carbonylverbindung der Formel (II) und Wasserstoff,

NH₂-CH₂-A-CH₂-NH₂ (I)

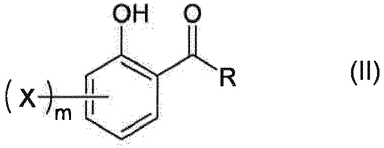

wobei
A für einen Phenylen-Rest oder Cyclohexylen-Rest steht,
R für einen Wasserstoff-Rest oder Alkyl-Rest mit 1 bis 8 C-Atomen steht, m für 0 oder 1 steht, und
X für einen Hydroxyl-Rest oder Methyl-Rest oder Methoxy-Rest steht, wobei das Molverhältnis zwischen dem Amin der Formel (I) und der Carbonylverbindung der Formel (II) im Bereich von 1/0.7 bis 1/1.2 liegt,
als kalthärtender Primer oder Klebstoff,
wobei das Reaktionsprodukt einen Gehalt an Amin der Formel (III) im Bereich von 30 bis 80 Gewichts-% aufweist.

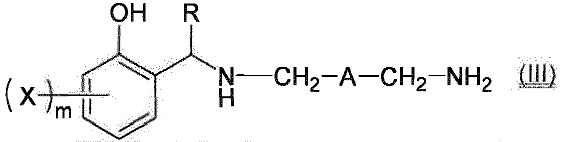

Als "Primer" wird eine härtbare Zusammensetzung bezeichnet, welche als Grundierung oder Voranstrich eingesetzt wird, indem sie flächig auf einen Untergrund bzw. ein Substrat appliziert und danach mit einer weiteren Schicht überdeckt wird. Bei der Aushärtung bildet der Primer einen Film, welcher den Untergrund bzw. das Substrat für die darauffolgende Schicht vorbereitet und als Haftbrücke dient. Die Schichtdicke des applizierten Primers liegt typischerweise im Mittel unterhalb von 0.5 mm.

Als "kalthärtend" wird ein Primer oder Klebstoff bezeichnet, welcher bei Umgebungstemperatur, insbesondere im Bereich von 5 bis 35 °C, verarbeitet und ausgehärtet werden kann.
Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.
Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.
Als "Aminwasserstoff" werden die Wasserstoffatome von primären und sekundären Aminogruppen bezeichnet.
Als "Aminwasserstoff-Equivalentgewicht" wird die Masse eines Amins oder einer Amin-haltigen Zusammensetzung, die ein Molequivalent Aminwasserstoff enthält, bezeichnet.
Als "Verdünner" wird eine in einem Epoxidharz lösliche und dessen Viskosität senkende Substanz bezeichnet, welche bei der Aushärtung chemisch nicht in das Epoxid-Polymer eingebunden wird.
Als "Viskosität" wird die dynamische Viskosität oder Scherviskosität bezeichnet, welche durch das Verhältnis zwischen der Schubspannung und der Scherrate (Geschwindigkeitsgefälle) definiert ist und wie in der Beschreibung bzw. den Ausführungsbeispielen beschrieben bestimmt wird.
Als "Raumtemperatur" wird eine Temperatur von 23 °C bezeichnet.

Bevorzugt wird die Epoxidharz-Zusammensetzung bei Umgebungstemperatur appliziert und ausgehärtet, bevorzugt bei einer Temperatur im Bereich von 5 bis 35 °C, besonders bevorzugt 10 bis 30 °C. Dies ist insbesondere für Anwendungen im Freien, auf Baustellen und in ungeheizten Industriehallen vorteilhaft.

Bevorzugt ist der Rest A in 1,3- oder in 1,4-Stellung substituiert. A steht also bevorzugt für 1,3-Phenylen, 1,4-Phenylen, 1,3-Cyclohexylen oder 1,4-Cyclohexylen. Diese Amine der Formel (I) sind besonders einfach zugänglich und besonders reaktiv.
Besonders bevorzugt steht A für 1,3-Phenylen oder 1,3-Cyclohexylen. Diese Amine der Formel (I) ermöglichen besonders niedrigviskose Reaktionsprodukte.

Bevorzugt ist das Amin der Formel (I) ausgewählt aus der Gruppe bestehend aus 1,3-Bis(aminomethyl)benzol und 1,3-Bis(aminomethyl)cyclohexan. Bevorzugt steht R für einen Wasserstoff-Rest oder für einen Alkyl-Rest mit 1 bis 4 C-Atomen, insbesondere Methyl, Ethyl oder Isopropyl.
Besonders bevorzugt steht R für einen Wasserstoff-Rest oder für einen Methyl-Rest.
Am meisten bevorzugt steht R für einen Wasserstoff-Rest.

Bevorzugt steht m für 0. Diese Carbonylverbindungen der Formel (II) ermöglichen besonders niedrigviskose Reaktionsprodukte.

Bevorzugte Carbonylverbindungen der Formel (II) sind 2-Hydroxybenzaldehyd (Salicylaldehyd), 2-Hydroxy-3-methylbenzaldehyd, 2-Hydroxy-5-methylbenz-aldehyd, 2-Hydroxy-3-methoxybenzaldehyd, 2-Hydroxy-4-methoxybenzaldehyd, 2-Hydroxy-5-methoxybenzaldehyd, 2'-Hydroxyacetophenon und 2-Hydroxy-4-methoxy-benzophenon. Diese Carbonylverbindungen sind entweder bei Raumtemperatur flüssig oder niedrigschmelzend mit einem Schmelzpunkt unterhalb von 60 °C, was eine einfache Handhabung ermöglicht.

Besonders bevorzugt ist die Carbonylverbindungen der Formel (II) ausgewählt aus der Gruppe bestehend aus Salicylaldehyd und 2'-Hydroxyacetophenon. Diese Carbonylverbindungen sind einfach erhältlich, bei Raumtemperatur flüssig und ermöglichen besonders niedrigviskose und besonders einfach herstellbare Reaktionsprodukte.
Am meisten bevorzugt ist Salicylaldehyd. Damit werden ganz besonders niedrigviskose und reaktive Reaktionsprodukte erhalten.

Bevorzugt liegt das Molverhältnis zwischen dem Amin der Formel (I) und der Carbonylverbindung der Formel (II) im Bereich von 1/0.8 bis 1/1.1, insbesondere 1/0.9 bis 1/1. Bei diesem Molverhältnis entstehen besonders gut verdünnende und besonders reaktive Reaktionsprodukte.

Die Umsetzung von mindestens einem Amin der Formel (I) mit mindestens einer Carbonylverbindung der Formel (II) und Wasserstoff wird auch als reduktive Alkylierung bezeichnet. Sie kann direkt mit molekularem Wasserstoff oder indirekt durch Wasserstoff-Transfer von anderen Reagentien erfolgen. Bevorzugt wird molekularer Wasserstoff verwendet. Die Reaktionsbedingungen werden vorteilhaft so gewählt, dass das Amin der Formel (I) grossteils nur einfach alkyliert wird und der aromatische Ring der Carbonylverbindung der Formel (II) nicht hydriert wird. Bevorzugt wird bei einem Wasserstoff-Druck von 5 bis 100 bar, einer Temperatur von 40 bis 120 °C, insbesondere 60 bis 100 °C, und in Anwesenheit eines geeigneten Katalysators gearbeitet. Als Katalysator bevorzugt sind Palladium auf Kohle (Pd/C), Platin auf Kohle (Pt/C), Adams-Katalysator oder Raney-Nickel, insbesondere Palladium auf Kohle. Die Umsetzung kann lösemittelfrei oder bevorzugt in einem Lösemittel, wie beispielsweise Ethanol oder Isopropanol, durchgeführt werden. Ein gegebenenfalls vorhandenes Lösemittel wird nach der Umsetzung bevorzugt entfernt, insbesondere mittels Destillation. 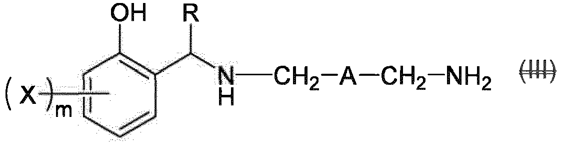 Die weiteren Bestandteile des Reaktionsprodukts bestehen mehrheitlich aus Amin der Formel (I) und mehrfach alkylierten Produkten, insbesondere N,N'-Dialkylierungsprodukten der Formel 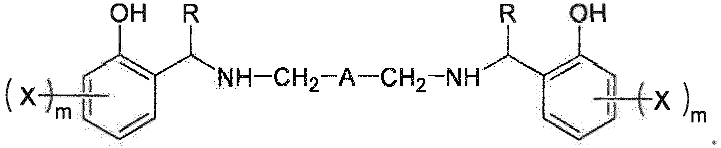 Bevorzugt umfasst die Epoxidharz-Zusammensetzung
- eine Harz-Komponente enthaltend mindestens ein Epoxidharz und
- eine Härter-Komponente enthaltend das Reaktionsprodukt aus der Umsetzung von mindestens einem Amin der Formel (I) mit mindestens einer Carbonylverbindung der Formel (II) und Wasserstoff, wie vorgängig beschrieben.

Dabei liegen die Harz- und die Härter-Komponente typischerweise in voneinander getrennten Gebinden vor und sind jede für sich lagerstabil. Sie werden erst unmittelbar vor der Applikation miteinander vermischt, so dass ihre Reaktivgruppen miteinander in Kontakt kommen und die Zusammensetzung aushärtet.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.
Als Epoxidharz besonders geeignet sind sogenannte Flüssigharze. Diese weisen eine Glasübergangstemperatur unterhalb von 25°C auf.
Ebenfalls möglich als Epoxidharz sind sogenannte Festharze, welche eine Glasübergangstemperatur oberhalb von 25°C aufweisen und sich zu bei 25°C schüttfähigen Pulvern zerkleinern lassen.

Geeignete Epoxidharze sind insbesondere aromatische Epoxidharze, insbesondere die Glycidylisierungsprodukte von:
- Bisphenol-A, Bisphenol-F oder Bisphenol-A/F, wobei A für Aceton und F für Formaldehyd steht, welche als Edukte zur Herstellung dieser Bisphenole dienten. Im Fall von Bisphenol-F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzkatechin;
- weiteren Bisphenolen oder Polyphenolen wie Bis(4-hydroxy-3-methylphenyl)methan, 2,2-Bis(4-hydroxy-3-methylphenyl)propan (Bisphenol-C), Bis-(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)heptan, 2,4-Bis(4-hydroxyphenyl)-2-methylbutan, 2,4-Bis-(3,5-dimethyl-4-hydroxyphenyl)-2-methylbutan, 1,1-Bis(4-hydroxyphenyl)-cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxy-naphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Kondensationsprodukten von Phenolen mit Formaldehyd, die unter sauren Bedingungen erhalten werden, wie Phenol-Novolaken oder Kresol-Novolaken;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylen-bis(1-methylethyliden)]bisanilin (Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, cyklischen oder offenkettigen di-, tri- oder tetrafunktionellen C₂-bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.
- Epoxidharze aus der Oxidation von Olefinen, wie insbesondere Vinylcylohexen, Dicyclopentadien, Cyclohexadien, Cyclododecadien, Cyclododecatrien, Isopren, 1,5-Hexadien, Butadien, Polybutadien oder Divinylbenzol.

Als Epoxidharz in der Harz-Komponente bevorzugt ist ein Flüssigharz auf der Basis eines Bisphenols, insbesondere ein Diglycidylether von Bisphenol-A, Bisphenol-F oder Bisphenol-A/F, wie sie kommerziell beispielsweise von Dow, Huntsman oder Momentive erhältlich sind. Diese Flüssigharze weisen eine für Epoxidharze niedrige Viskosität und im ausgehärteten Zustand gute Eigenschaften als Beschichtung auf. Sie können Anteile von Bisphenol A-Festharz oder Phenol-Novolaken enthalten.

Die Harz-Komponente kann einen Reaktivverdünner, insbesondere einen mindestens eine Epoxidgruppe aufweisenden Reaktivverdünner, enthalten. Als Reaktivverdünner geeignet sind insbesondere die Glycidylether von ein- oder mehrwertigen Phenolen oder aliphatischen oder cycloaliphatischen Alkoholen, wie insbesondere die bereits genannten Polyglycidylether von Di- oder Polyolen, oder weiterhin Phenylglycidylether, Kresylglycidylether, Guaiacolglycidylether, 4-Methoxyphenylglycidylether, p-n-Butylphenylglycidylether, p-tert.Butylphenylglycidylether, 4-Nonylphenylglycidylether, 4-Dodecylphenylglycidylether, Cardanolglycidylether, Benzylglycidylether, Allylglycidylether, Butylglycidylether, Hexylglycidylether, 2-Ethylhexylglycidylether, oder Glycidylether von natürlichen Alkoholen wie insbesondere C₈- bis C₁₀-Alkylglycidylether oder C₁₂-bis C₁₄-Alkylglycidylether. Die Zugabe eines Reaktivverdünners zum Epoxidharz bewirkt eine Reduktion der Viskosität, und/oder eine Reduktion der Glasübergangstemperatur und/oder der mechanischen Werte.

Die Epoxidharz-Zusammensetzung enthält bevorzugt zusätzlich mindestens einen Verdünner und/oder mindestens einen Beschleuniger und/oder mindestens ein weiteres Amin.

Das weitere Amin ist dabei insbesondere kein Amin der Formel (III) und kein im Reaktionsprodukt enthaltenes Edukt oder Nebenprodukt.

Als Verdünner geeignet ist insbesondere Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykol-monoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie zum Beispiel Solvesso®-Typen (von Exxon), Alkylphenole wie tert.Butylphenol, Nonylphenol, Dodecylphenol oder Cardanol (aus Cashew-Schalen-Öl, enthaltend als Hauptbestandteile 3-(Pentadeca-8-enyl)phenol, 3-(Pentadeca-8,11-dienyl)phenol und 3-(Pentadeca-8,11,14-trienyl)phenol), erhältlich beispielsweise als Cardolite® NC-700 oder Cardolite® NX-2026 (beide von Cardolite), styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide.
Bevorzugte Verdünner sind Benzylalkohol, styrolisiertes Phenol, ethoxyliertes Phenol, phenolgruppenhaltige aromatische Kohlenwasserstoffharze, insbesondere die Novares®-Typen LS 500, LX 200, LA 300 oder LA 700 (von Rütgers), oder Cardanol.

In einer bevorzugten Ausführungsform enthält die Epoxidharz-Zusammensetzung mindestens einen Verdünner ausgewählt aus der Gruppe bestehend aus Benzylalkohol, styrolisiertem Phenol, ethoxyliertem Phenol, phenolgruppenhaltigen aromatische Kohlenwasserstoffharzen und Cardanol. Eine solche Epoxidharz-Zusammensetzung ist besonders geeignet für die Verwendung als Primer.

Es kann vorteilhaft sein, eine Kombination von zwei oder mehreren Verdünnern einzusetzen.

Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; tertiäre Amine wie insbesondere 1,4-Di-azabicyclo[2.2.2]octan, Benzyldimethylamin, α-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]undec-7-en, Guanidine wie insbesondere 1,1,3,3-Tetramethylguanidin, Phenole, insbesondere Bisphenole, Phenol-Harze oder tertiäre Aminogruppen aufweisende Mannich-Basen wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen. Als Beschleuniger bevorzugt sind Säuren, tertiäre Amine oder tertiäre Aminogruppen aufweisende Mannich-Basen.
Am meisten bevorzugt ist Salicylsäure oder 2,4,6-Tris(dimethylaminomethyl)-phenol oder eine Kombination davon.

Weitere Amine sind bevorzugt ein Bestandteil der Härter-Komponente.
Als weiteres Amin geeignet sind Polyamine, welche mindestens zwei, insbesondere mindestens drei, gegenüber Epoxidgruppen reaktive Aminwasserstoffe aufweisen, insbesondere die Folgenden:
- Polyamine mit einer primären Aminogruppe, insbesondere N,N-Dimethylaminopropylamin (DMAPA) oder N-(3-Dimethylaminopropyl)-1,3-propylendiamin (DMAPAPA);
- Polyamine mit ein oder zwei sekundären Aminogruppen, insbesondere Produkte aus der reduktiven Alkylierung von primären aliphatischen Polyaminen mit Aldehyden oder Ketonen, welche nicht der Formel (III) entsprechen, wie insbesondere N-Benzyl-1,2-ethandiamin, N,N'-Dibenzyl-1,2-ethandiamin, N¹-Benzyl-1,2-propandiamin oder N²-Benzyl-1,2-propandiamin oder beliebige Gemische dieser Isomeren, N,N'-Dibenzyl-1,2-propandiamin, N-Benzyl-1,3-bis(aminomethyl)benzol, N,N'-Dibenzyl-1,3-bis(aminomethyl)benzol, N-Benzyl-1,3-bis(aminomethyl)cyclohexan, N,N'-Dibenzyl-1,3-bis(aminomethyl)-cyclohexan, N-2-Ethylhexyl-1,3-bis(aminomethyl)benzol, N,N'-Bis(2-ethylhexyl)-1,3-bis(aminomethyl)benzol, oder partiell styrolisierte Polyamine wie insbesondere styrolisiertes 1,3-Bis(aminomethyl)benzol (erhältlich als Gaskamine® 240 von Mitsubishi Gas Chemical);
- aliphatische, cycloaliphatische oder arylaliphatische primäre Diamine, insbesondere 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2(4),4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, Bis(4-aminocyclohexyl)methan (H₁₂-MDA), Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (Isophorondiamin oder IPDA), 2- oder 4-Methyl-1,3-diaminocyclohexan oder Mischungen davon, 1,3-Bis(aminomethyl)cyclohexan, 1,4-Bis(aminomethyl)cyclohexan, 2,5(2,6)-Bis(aminomethyl)bicyclo[2.2.1]heptan (NBDA), 3(4),8(9)-Bis(aminomethyl)tricyclo-[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol (MXDA) oder 1,4-Bis(aminomethyl)benzol;
- Ethergruppen-haltige aliphatische primäre Di- oder Triamine, insbesondere Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Di-oxadodecan-3,10-diamin, 4,7,10-Trioxatridecan-1,13-diamin oder höhere Oligomere dieser Diamine, Bis(3-aminopropyl)polytetrahydrofurane oder andere Polytetrahydrofurandiamine, cycloaliphatische Ethergruppen-haltige Diamine aus der Propoxylierung und nachfolgenden Aminierung von 1,4-Dimethylolcyclohexan, erhältlich insbesondere als Jeffamine® RFD-270 (von Huntsman), oder Polyoxyalkylendi- oder -triamine, welche typischerweise Produkte aus der Aminierung von Polyoxyalkylendi- oder -triolen darstellen und beispielsweise erhältlich sind unter dem Namen Jeffamine® (von Huntsman), unter dem Namen Polyetheramine (von BASF) oder unter dem Namen PC Amine® (von Nitroil). Insbesondere geeignete Polyoxyalkylendi- oder -triamine sind Jeffamine® D-230, Jeffamine® D-400, Jeffamine® D-2000, Jeffamine® EDR-104, Jeffamine® EDR-148, Jeffamine® EDR-176, Jeffamine® T-403, Jeffamine® T-3000, Jeffamine® T-5000, oder entsprechende Amine von BASF oder Nitroil;
- sekundäre Aminogruppen aufweisende Polyamine mit zwei primären aliphatischen Aminogruppen, wie insbesondere 3-(2-Aminoethyl)aminopropylamin, Bis(hexamethylen)triamin (BHMT), Diethylentriamin (DETA), Triethylentetramin (TETA), Tetraethylenpentamin (TEPA), Pentaethylenhexamin (PEHA) oder höhere Homologe linearer Polyethylenamine wie Polyethylenpolyamin mit 5 bis 7 Ethylenamin-Einheiten (sogenanntes "higher ethylenepolyamine", HEPA), Produkte aus der mehrfachen Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin oder N,N'-Bis(3-amino-1-ethylpropyl)-2-methyl-1,5-pentandiamin;
- aliphatische, cycloaliphatische oder arylaliphatische primäre Triamine, insbesondere 4-Aminomethyl-1,8-octandiamin, 1,3,5-Tris(aminomethyl)benzol, 1,3,5-Tris(aminomethyl)cyclohexan, Tris(2-aminoethyl)amin, Tris(2-amino-propyl)amin oder Tris(3-aminopropyl)amin;
- aromatische Polyamine, wie insbesondere m- und p-Phenylendiamin, 4,4'-, 2,4' und/oder 2,2'-Diaminodiphenylmethan, 3,3'-Dichloro-4,4'-diaminodiphenylmethan (MOCA), 2,4- und/oder 2,6-Toluylendiamin, Mischungen von 3,5-Dimethylthio-2,4- und -2,6-toluylendiamin (erhältlich als Ethacure® 300 von Albermarle), Mischungen von 3,5-Diethyl-2,4- und -2,6-toluylendiamin (DETDA, erhältlich als Ethacure® 100 von Albermarle), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-(5,5'-methylendianthranilat), 1,3-Propylen-bis(4-aminobenzoat), 1,4-Butylen-bis(4-aminobenzoat), Polytetramethylenoxid-bis(4-aminobenzoat) (erhältlich als Versalink® von Air Products), 1,2-Bis(2-aminophenylthio)ethan, 2-Methylpropyl-(4-chloro-3,5-diaminobenzoat) oder tert.Butyl-(4-chloro-3,5-diamino-benzoat);
- Polyamidoamine, insbesondere Umsetzungsprodukte aus einer ein- oder mehrwertigen Carbonsäure, beziehungsweise deren Ester oder Anhydride, insbesondere einer Dimerfettsäure, mit einem im stöchiometrischen Überschuss eingesetzten aliphatischen, cycloaliphatischen oder aromatischen Polyamin, insbesondere einem Polyalkylenamin wie beispielsweise DETA oder TETA, insbesondere die kommerziell erhältlichen Polyamidoamine Versamid® 100, 125, 140 oder 150 (von Cognis), Aradur® 223, 250 oder 848 (von Huntsman), Euretek®3607 oder 530 (von Huntsman) oder Beckopox® EH 651, EH 654, EH 655, EH 661 oder EH 663 (von Cytec);
- oder Addukte von Polyaminen mit Epoxiden oder Epoxidharzen, insbesondere Addukte mit Diepoxiden im Molverhältnis von ungefähr 2/1, oder Addukte mit Monoepoxiden im Molverhältnis von ungefähr 1/1, oder Umsetzungsprodukte aus Polyaminen und Epichlorhydrin, insbesondere jenes von 1,3-Bis(aminomethyl)benzol, kommerziell erhältlich als Gaskamine® 328 (von Mitsubishi Gas Chemical).

Es kann vorteilhaft sein, wenn der Härter eine Kombination aus zwei oder mehr weiteren Aminen enthält.

Als weiteres Amin bevorzugt sind Polyamine mit einer primären und einer sekundären Aminogruppe, welche nicht der Formel (III) entsprechen, insbesondere N-Benzyl-1,2-ethandiamin, N¹-Benzyl-1,2-propandiamin oder N²-Benzyl-1,2-propandiamin oder beliebige Gemische dieser Isomeren, N-Benzyl-1,3-bis-(aminomethyl)benzol oder N-Benzyl-1,3-bis(aminomethyl)cyclohexan. Solche Amine weisen eine hohe Verdünnungswirkung auf.

Als weiteres Amin bevorzugt sind weiterhin Polyamine mit einem Molekulargewicht von mindestens 120 g/mol, bevorzugt TMD, H₁₂-MDA, IPDA, 2- oder 4-Methyl-1,3-diaminocyclohexan oder Mischungen davon, 1,3-Bis(aminomethyl)cyclohexan, 1,4-Bis(aminomethyl)cyclohexan, NBDA, MXDA, BHMT, TETA, TEPA, PEHA, DPTA oder N4-Amin. Solche Amine weisen eine hohe Verdünnungswirkung und eine hohe Reaktivität auf, ohne dass sie allzu flüchtig sind.

Als weiteres Amin bevorzugt sind weiterhin DMAPA oder DMAPAPA. Diese Amine weisen eine hohe Verdünnungswirkung und eine hohe Reaktivität auf.

Als weiteres Amin bevorzugt sind weiterhin mindestens drei Aminwasserstoffe aufweisende Addukte aus mindestens einem Polyamin mit 2 bis 12 C-Atomen und mindestens einem Epoxid.
Als Epoxid für ein solches Addukt bevorzugt sind Monoepoxide, insbesondere aromatische Monoepoxide, insbesondere Kresylglycidylether, tert.Butylphenylglycidylether oder der Glycidylether von Cardanol. Besonders bevorzugt ist Kresylglycidylether. Als Kresylglycidylether geeignet sind alle isomeren Kresylglycidylether oder Gemische davon, insbesondere kommerziell erhältiche Typen wie insbesondere Araldite® DY-K (von Huntsman), Polypox™ R6 (von Dow), Heloxy™ KR (von Hexion) oder Erisys® GE-10 (von CVC Spec. Chem.) Als Epoxid für ein solches Addukt bevorzugt sind weiterhin Diepoxide, insbesondere aromatische Diepoxide, insbesondere Bisphenol-A- oder Bisphenol-F-oder Bisphenol-A/F-Diglycidylether oder Resorcinol-Diglycidylether, insbesondere kommerziell erhältliche Flüssigharze.

Als weiteres Amin besonders bevorzugt ist N¹-Benzyl-1,2-propandiamin oder N²-Benzyl-1,2-propandiamin oder beliebige Gemische dieser Isomeren.
Als weiteres Amin besonders bevorzugt ist weiterhin N-Benzyl-1,3-bis(aminomethyl)benzol oder N-Benzyl-1,3-bis(aminomethyl)cyclohexan.

Die Härter-Komponente kann weitere gegenüber Epoxidgruppen reaktive Substanzen enthalten, beispielsweise Monoamine wie Hexylamin oder Benzylamin, oder Mercaptogruppen aufweisende Verbindungen, insbesondere die Folgenden:
- flüssige Mercaptan-terminierte Polysulfid-Polymere, bekannt unter dem Markennamen Thiokol® (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 oder LP-2; sowie weiterhin bekannt unter dem Markennamen Thioplast® (von Akzo Nobel), insbesondere die Typen G 10, G 112, G 131, G 1, G 12, G 21, G 22, G 44 oder G 4;
- Mercaptan-terminierte Polyoxyalkylen-Ether, erhältlich beispielsweise durch Umsetzung von Polyoxyalkylendi- oder -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure® (von Cognis), insbesondere die Typen WR-8, LOF oder 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri(3-mercaptopropionat) oder Glykoldi-(3-mercaptopropionat), oder Veresterungsprodukte von Polyoxyalkylendiolen oder -triolen, ethoxyliertem Trimethylolpropan oder Polyester-Diolen mit Thiocarbonsäuren wie Thioglykolsäure oder 2- oder 3-Mercaptopropionsäure; oder
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) oder Ethandithiol.

Die Härter-Komponente enthält bevorzugt einen niedrigen Gehalt an chemisch nicht ins Epoxid-Polymer einbindbaren toxischen Phenol-Verbindungen, insbesondere solchen ausgewählt aus der Gruppe bestehend aus Phenol, Kresol, Resorcin, tert.Butylphenol, Nonylphenol und Dodecylphenol. Bevorzugt enthält sie weniger als 5 Gewichts-%, besonders bevorzugt weniger als 1 Gewichts-%, insbesondere weniger als 0.1 Gewichts-%, solcher Phenol-Verbindungen. Am meisten bevorzugt ist die Härter-Komponente ganz frei von solchen Phenol-Verbindungen. Eine solche Epoxidharz-Zusammensetzung ist toxikologisch besonders vorteilhaft.

Die Härter-Komponente enthält weiterhin bevorzugt einen niedrigen Gehalt an Aminen mit einem Molekulargewicht unterhalb von 120 g/mol. Bevorzugt enthält sie weniger als 2 Gewichts-%, insbesondere weniger als 1 Gewichts-%, Amine mit einem Molekulargewicht unterhalb von 120 g/mol. Eine solche Epoxidharz-Zusammensetzung ist toxikologisch und geruchlich besonders vorteilhaft.

Die Epoxidharz-Zusammensetzung enthält bevorzugt soviel des beschriebenen Reaktionsprodukts, dass 30 bis 100%, bevorzugt 50 bis 100%, insbesondere 70 bis 100%, der gesamten Aminwasserstoffe der Härter-Komponente aus dem Reaktionsprodukt stammen.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner oder Extender, wie insbesondere die bereits genannten Verdünner;
- Reaktivverdünner, insbesondere Epoxidgruppen aufweisende Reaktivverdünner, wie sie vorgängig erwähnt wurden, epoxidierte natürliche Öle wie Sojaöl, Leinöl oder Palmkernöl, oder Acetoacetatgruppen aufweisende Verbindungen, insbesondere acetoacetylierte Polyole, Butyrolakton, Carbonate, Aldehyde, sowie weiterhin Isocyanate oder Reaktivgruppen-aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere, Sulfonamid-modifizierte Melamine oder gereinigte Montan-Wachse;
- anorganische oder organische Füllstoffe, insbesondere gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid und/oder Eisenoxide;
- die vorgenannten Beschleuniger;
- Rheologie-Modifizierer, insbesondere Verdicker oder Antiabsetzmittel;
- Haftverbesserer, insbesondere Organoalkoxysilane;
- Stabilisatoren gegen Oxidation, Wärme, Licht oder UV-Strahlung;
- flammhemmende Substanzen, insbesondere Aluminiumhydroxid (ATH), Magnesiumdihydroxid (MDH), Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat oder Bisphenol-A-bis(diphenylphosphat), Tris(chloroethyl)phosphat, Tris(chloropropyl)phosphat oder Tris(dichloroisopropyl)phosphat, Tris[3-bromo-2,2-bis-(bromomethyl)propyl]phosphat, Tetrabromo-Bisphenol-A, Bis(2,3-dibromo-propylether) von Bisphenol A, bromierte Epoxidharze, Ethylen-bis(tetrabro-mophthalimid), Ethylen-bis(dibromonorbornandicarboximid), 1,2-Bis(tribro-mophenoxy)ethan, Tris(2,3-dibromopropyl)isocyanurat, Tribromophenol, Hexabromocyclododecan, Bis(hexachlorocyclopentadieno)cyclooctan oder Chlorparaffine;
- oberflächenaktive Substanzen, insbesondere Netzmittel, Verlaufsmittel, Entlüftungsmittel oder Entschäumer;
- Biozide, wie beispielsweise Algizide, Fungizide oder das Pilzwachstum hemmende Substanzen.

Bei der Verwendung als Klebstoff enthält die Epoxidharz-Zusammensetzung bevorzgt Füllstoffe und/oder Pigmente und/oder Beschleuniger.

In der Epoxidharz-Zusammensetzung liegt das Verhältnis der Anzahl von gegenüber Epoxidgruppen reaktiven Gruppen gegenüber der Anzahl Epoxidgruppen bevorzugt im Bereich von 0.5 bis 1.5, insbesondere 0.8 bis 1.2.

Die Komponenten der Epoxidharz-Zusammensetzung werden jeweils in einem eigenen Gebinde gelagert. Weitere Bestandteile der Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche, eine Tube oder eine Flasche. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der Epoxidharz-Zusammensetzung werden die Harz- und die Härter-Komponente kurz vor oder während der Applikation miteinander vermischt. Das Mischungsverhältnis zwischen den Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.
Die Vermischung der Komponenten erfolgt mittels eines geeigneten Verfahrens. Die Vermischung kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, beispielsweise einem verlangsamten oder unvollständigen Aufbau der Haftung, kommen kann. Die Vermischung erfolgt insbesondere bei Umgebungstemperatur, welche typischerweise im Bereich von etwa 5 bis 50°C, bevorzugt bei etwa 10 bis 30°C, liegt.
Beim Vermischen der Komponenten beginnt die Aushärtung durch chemische Reaktion. Dabei reagieren die Epoxidgruppen mit den Aminwasserstoff tragenden Aminogruppen und gegebenenfalls vorhandenen weiteren gegenüber Epoxidgruppen reaktiven Gruppen unter Ringöffnung zu Aminoalkohol-Einheiten. Weitere Epoxidgruppen reagieren mit sich selbst unter anionischer Polymerisation. Als Ergebnis dieser Reaktionen härtet die Zusammensetzung zu einem vernetzten Material aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.
Die Aushärtung erfolgt insbesondere bei Umgebungstemperatur. Sie erstreckt sich typischerweise über einige Stunden bis Tage, bis sie unter den gegebenen Bedingungen weitgehend abgeschlossen ist. Wichtige Einflussgrössen sind dabei die Temperatur, die Stöchiometrie und die Gegenwart von Beschleunigern.
Aus der Aushärtung der Epoxidharz-Zusammensetzung wird ein ausgehärteter Primer oder Klebstoff erhalten.

Die Applikation der Epoxidharz-Zusammensetzung erfolgt auf mindestens ein Substrat, wobei die Folgenden besonders geeignet sind:
- Glas, Glaskeramik, Beton, Mörtel, Zement-Estrich, Anhydrit-Estrich, Magnesia-Estrich, Backstein, Ziegel, Gips oder Natursteine wie Granit oder Marmor;
- Metalle oder Legierungen wie Aluminium, Eisen, Stahl oder Buntmetalle, oder oberflächenveredelte Metalle oder Legierungen wie verzinkte oder verchromte Metalle;
- Holz, mit Harzen, beispielsweise Phenol-, Melamin- oder Epoxidharzen, gebundene Holzwerkstoffe oder weitere sogenannte Polymer-Composites;
- Kunststoffe, insbesondere Hart- oder Weich-PVC, ABS, Polycarbonat (PC), Polyamid (PA), Polyester, PMMA, Epoxidharze, PUR, POM, PO, PE, PP, EPM oder EPDM, wobei die Kunststoffe gegebenenfalls mittels Plasma, Corona oder Flammen oberflächenbehandelt sind;
- Faserverstärkte Kunststoffe, wie Kohlefaser-verstärkte Kunststoffe (CFK), Glasfaser-verstärkte Kunststoffe (GFK) oder Sheet Moulding Compounds (SMC);
- beschichtete Substrate, wie pulverbeschichtete Metalle oder Legierungen;
- Farben oder Lacke.
Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten und/oder Abblasen, sowie weiterhin Behandeln mit Reinigern oder Lösemitteln oder das Aufbringen eines Haftvermittlers, einer Haftvermittlerlösung oder eines Primers.
Poröse mineralische Substrate sind bevorzugt so vorbehandelt, dass eine offenporige, weitgehend staubfreie Oberfläche ohne Zementhaut vorliegt.

Ein weiterer Gegenstand der Erfindung ist ein Primer, umfassend
- eine Harz-Komponente enthaltend mindestens ein Epoxidharz, wie vorgängig beschrieben, und
- eine Härter-Komponente enthaltend mindestens ein Reaktionsprodukt aus der Umsetzung von mindestens einem Amin der Formel (I) mit mindestens einer Carbonylverbindung der Formel (II) und Wasserstoff, wie vorgängig beschrieben.

Der Primer weist insbesondere eine Viskosität bei 20 °C von höchstens 5 Pa·s auf. Bevorzugt liegt die Viskosität bei 20 °C in Bereich von 0.3 bis 4 Pa·s, insbesondere 0.5 bis 3 Pa·s.
Die Viskosität wird bestimmt, indem die Komponenten des Primers durch Rühren oder Schütteln gut vermischt werden und 5 Minuten nach dem Vermischen auf einem auf 20 °C thermostatisierten Kegel-Platten-Viskosimeter mit Kegeldurchmesser 50 mm, Kegelwinkel 1°, Kegelspitze-Platten-Abstand 0.05 mm die Viskosität bei einer Scherrate von 10 s⁻¹ gemessen wird.
Der Primer ist dadurch insbesondere geeignet zur Vorbehandlung von porösen mineralischen Substraten, da er dünnflüssig genug ist, um beim Applizieren auf ein poröses mineralisches Substrat bei Umgebungstemperaturen so gut in das Substrat einzudringen, dass dessen Poren mit Primer gefüllt und verschlossen werden.

Bevorzugt enthält der Primer zusätzlich zum beschriebenen Reaktionsprodukt eine verdünnende Substanz, insbesondere einen Verdünner oder ein weiteres Amin.

Als weiteres Amin geeignet ist insbesondere N¹-Benzyl-1,2-propandiamin oder N²-Benzyl-1,2-propandiamin oder beliebige Gemische dieser Isomeren, N-Benzyl-1,3-bis(aminomethyl)benzol, N-Benzyl-1,3-bis(aminomethyl)cyclohexan, TMD, H₁₂-MDA, IPDA, 2- oder 4-Methyl-1,3-diaminocyclohexan oder Mischungen davon, 1,3-Bis(aminomethyl)cyclohexan, 1,4-Bis(aminomethyl)cyclohexan, NBDA, MXDA, BHMT, TETA, TEPA, PEHA, DPTA, N4-Amin, DMAPA oder DMAPAPA.

Bevorzugt enthält der Primer mindestens einen Verdünner ausgewählt aus der Gruppe bestehend aus Benzylalkohol, styrolisiertem Phenol, ethoxyliertem Phenol, phenolgruppenhaltigen aromatische Kohlenwasserstoffharzen und Cardanol.

Bevorzugt enthält der Primer 1.5 bis 30 Gewichts-%, insbesondere 3 bis 20 Gewichts-%, eines solchen Verdünners.
Bevorzugt ist der Verdünner zumindest teilweise ein Bestandteil der Härter-Komponente.
Bevorzugt enthält die Härter-Komponente 5 bis 60 Gewichts-%, insbesondere 10 bis 50 Gewichts-%, Verdünner.

Als Verdünner besonders bevorzugt ist Benzylalkohol. Benzylalkohol hat eine besonders hohe Verdünnungswirkung und ist besonders gut verträglich mit dem Epoxid-Polymer.
Als Verdünner weiterhin besonders bevorzugt ist Cardanol. Damit wird eine besonders schnelle Aushärtung erhalten. Cardanol ist ein nachwachsender Rohstoff und stellt eine besonders wenig toxische Phenol-Verbindung dar. Es wird aus Cashew-Schalen-Öl gewonnen und enthält als Hauptbestandteile 3-(Petadeca-8-enyl)phenol, 3-(Pentadeca-8,11-dienyl)phenol und 3-(Pentadeca-8,11,14-trienyl)phenol).

Bevorzugt enthält die Härter-Komponente des Primers weniger als 0.1 Gewichts-% Phenol-Verbindungen ausgewählt aus der Gruppe bestehend aus Phenol, Kresol, Resorcin, tert.Butylphenol, Nonylphenol und Dodecylphenol. Besonders bevorzugt ist die Härter-Komponente ganz frei von solchen Phenol-Verbindungen. Ein solcher Primer ist toxikologisch besonders vorteilhaft.

Primer werden oft auf grosse Flächen appliziert, beispielsweise als Grundierung für flüssig applizierte Fussboden-Beschichtungen. Dabei ist eine sehr schnelle Aushärtung auch bei kalter Umgebungstemperatur gefordert, damit der Primer für die folgenden Arbeitsschritte möglichst bald begehbar ist. Um begehbar zu sein, muss der Primer nicht nur leicht angehärtet sein, sondern eine nichtklebrige, belastbare Oberfläche aufweisen. Zudem sind der Verarbeiter und die Umgebung bei grossflächiger Anwendung den im Primer enthaltenden, chemisch nicht einbindbaren Substanzen besonders stark ausgesetzt. Dadurch sind die Anforderungen an die Aushärtungsgeschwindigkeit und die Schwierigkeiten mit toxischen Phenol-Verbindungen in Primer-Anwendungen besonders hoch.

In einer bevorzugten Ausführungsform wird der Primer bei einer Temperatur unterhalb von 20 °C appliziert und/oder ausgehärtet.
Insbesondere kann er unterhalb von 15 °C oder 10 °C appliziert und/oder ausgehärtet werden, wobei er dünnflüssig genug für die Vorbehandlung von porösen mineralischen Substraten ist und schnell und störungsfrei aushärtet.

Der Primer weist nach dem Vermischen der Komponenten eine fliessfähige Konsistenz mit guten Verlaufseigenschaften auf. Bei der Applikation wird der frisch vermischte, noch flüssige Primer innerhalb seiner Offenzeit auf eine ebene oder leicht geneigte Fläche appliziert, typischerweise indem er mittels Bürste, Pinsel, Rolle oder Rakel so aufgetragen wird, dass der Untergrund bzw. das Substrat gut benetzt und gleichmässig bedeckt wird.
Als "Offenzeit" oder auch "Topfzeit" wird dabei die Zeitspanne zwischen dem Vermischen der Komponenten und dem Zeitpunkt, an dem der Primer nicht mehr ordnungsgemäss applizierbar ist, bezeichnet. Ein typisches Mass für das Ende der Topfzeit ist das Erreichen eines bestimmten Viskositätswerts. Ebenfalls möglich ist es, den vermischten Primer mittels einer geeigneten Apparatur auf das Substrat zu sprühen bzw. zu spritzen. Dies kann mit Hilfe von Luft oder mittels einer Airless-Spritzeinrichtung erfolgen.

Im Fall eines porösen Substrats wird der Primer typischerweise in einer solchen Menge aufgetragen, dass der Primer nach der Aushärtung einen geschlossenen Film auf der Oberfläche bildet und dabei die Poren verschliesst. Bei besonders porösen Substraten kann es vorteilhaft sein, den Primer in zwei Arbeitsgängen aufzutragen, um eine porenfreie, geschlossene Oberfläche zu erhalten.
Im Fall eines nicht-porösen Substrats wird der Primer typischerweise in einer Schichtdicke im Bereich von 0.1 bis 0.5 mm aufgetragen.

Falls gewünscht, kann der Primer vor der Verarbeitung mit Füllstoff oder Fasern oder anderen sogenannten Stellmitteln versetzt werden, damit er eine verdickte, leicht thixotrope Konsistenz aufweist und bei der Applikation auf geneigte Flächen oder über Kopf weniger stark abfliesst.

Das poröse mineralische Substrat ist insbesondere ein Substrat auf der Basis von Zement, Anhydrit (Calciumsulfat) oder Magnesia (Magnesit), wie insbesondere Beton, Mörtel, Zement-Estrich, Anhydrit-Estrich oder Magnesia-Estrich, oder Backstein, Ziegel, Gips oder Naturstein.
Bevorzugt ist ein Substrat auf der Basis von Zement, Anhydrit oder Magnesia. Diese Substrate bilden typischerweise den Untergrund für flüssig applizierte Bodenbeschichtungen in Bauwerken, wobei die Schwierigkeit besteht, dass die Aushärtung der Bodenbeschichtung durch Feuchtigkeit im Untergrund gestört werden kann und durch Poren im Substrat unschöne Krater und Blasen auf der Oberfläche der ausgehärteten Beschichtung auftreten können. Die Verwendung des erfindungsgemässen Primers als Grundierung auf diesen Substraten verschliesst die Poren, verdichtet den Untergrund und wirkt als Haftbrücke und Sperre gegen Feuchtigkeit von unten, wodurch der Untergrund optimal vorbereitet ist für die Überschichtung mit einer härtbaren Polymerzusammensetzung.

Weiterhin kann der Primer als Grundierung für Metalle, Kunststoffe oder Holz verwendet werden, insbesondere für Eisen, Stahl oder Beschichtungen aus Kunststoff, wobei Eisen und Stahl verzinkt, verchromt oder beschichtet oder als Legierung vorhanden sein können.

Ein weiterer Gegenstand der Erfindung ist ein Schicht-Aufbau, umfassend
- mindestens eine Schicht des erfindungsgemässen Primers, insbesondere in einer solchen Menge, dass der Primer nach der Aushärtung einen geschlossenen Film bildet und vorhandene Poren verschliesst,
- mindestens eine Schicht einer härtbaren Polymerzusammensetzung,
- gegebenenfalls eine Deckschicht und/oder Versiegelung.

Bevorzugt stellt der Schicht-Aufbau ein Beschichtungs-System dar, wobei die härtbare Polymerzusammensetzung eine Kunststoff-Beschichtung ist und insbesondere in einer Schichtdicke im Bereich von 0.5 bis 3 mm vorhanden ist.

Die Kunststoff-Beschichtung ist dabei insbesondere
- eine einkomponentige oder eine zwei- oder mehrkomponentige Polyurethan- oder Polyharnstoff-Beschichtung,
- eine zwei- oder mehrkomponentige Epoxidharz-Beschichtung,
- eine einkomponentige oder zwei- oder mehrkomponentige Beschichtung auf Basis eines Silangruppen aufweisenden Polymers, welche gegebenenfalls zusätzlich ein Epoxidharz enthält,
- eine wässrige Beschichtung basierend auf mindestens einer Kunststoff-Dispersion enthaltend Polymere auf der Basis von Polyurethan, Acrylat, StyrolButadien, PVA oder Copolymeren davon,
- eine zwei- oder mehrkomponentige wässrige Epoxidharz-Beschichtung umfassend einen wasserverdünnbaren Härter und eine Epoxidharz-Emulsion, oder
- eine zwei- oder mehrkomponentige wässrige Polyurethanbeschichtung umfassend eine Polyol-Emulsion und eine emulgierbare Isocyanatkomponente.

Dabei kann die Kunststoff-Beschichtung in einer oder mehreren Schichten appliziert werden. Typischerweise wird sie in einer oder insbesondere in zwei Schichten appliziert.

Ebenfalls möglich ist ein Schichtaufbau bestehend aus mehreren Schichten von sich unterscheidenden Kunststoff-Beschichtungen.

Als "Versiegelung" wird eine transparente oder pigmentierte, hochwertige Beschichtung bezeichnet, welche als oberste dünne Schicht auf eine Beschichtung appliziert wird. Sie schützt und veredelt deren Oberfläche und verschliesst noch vorhandene Poren. Die Schichtdicke einer Versiegelung (im trockenen Zustand) liegt typischerweise im Bereich von 0.03 bis 0.3 mm.

Das Beschichtungs-System wird bevorzugt verwendet als Bodenbelag bzw. Bodenbeschichtung für Innenräume wie Büros, Industriehallen, Turnhallen oder Kühlräume, oder im Aussenbereich für Balkone, Terrassen, Parkdecks, Brücken, Dächer, Sport- oder Spielplätze, als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden, oder als Abdichtung, insbesondere zum Abdichten von Brücken oder Dächern.
Bei der Verwendung als Brückenabdichtung kann das Beschichtungs-System zusätzlich mit einem weiteren geeigneten Belag, insbesondere Gussasphalt oder Beton, überbeschichtet sein.
Bei der Verwendung als Dachabdichtung kann das Beschichtungs-System zusätzlich mit Kies und/oder Erdreich überschichtet sein.

Besonders bevorzugt stellt das Beschichtungs-System einen Boden oder eine Schutzbeschichtung oder eine Wasserabdichtung dar.

Der Schicht-Aufbau kann auch eine Verklebung darstellen, wobei die härtbare Polymerzusammensetzung ein Klebstoff ist und eine Deckschicht in Form eines verklebten Substrats vorhanden ist.
Ein Beispiel eines solchen Schicht-Aufbaus ist ein verklebter Parkettboden, wobei beispielsweise ein Zement-Estrich mit dem erfindungsgemässen Primer grundiert ist und Parkett-Elemente mittels einem Klebstoff, beispielsweise einem elastischen Polyurethanklebstoff, direkt auf den vorbehandelten Zement-Estrich aufgeklebt sind.

Ein weiterer Gegenstand der Erfindung ist ein Verfahren zum Beschichten oder Verkleben eines porösen mineralischen Substrats, umfassend die Schritte
(i) Applizieren des vorgängig beschriebenen Primers auf das poröse mineralische Substrat, insbesondere in einer zum Verschliessen der Poren ausreichenden Menge,
(ii) Aushärten des applizierten Primers bei Umgebungstemperatur, insbesondere mindestens bis zur Begehbarkeit,
(iii) Applizieren von mindestens einer Schicht einer härtbaren Polymerzusammensetzung auf den applizierten und zumindest teilweise ausgehärteten Primer.

Dabei wird der Primer insbesondere bei einer Temperatur unterhalb von 20 °C appliziert und/oder ausgehärtet.

Bevorzugt ist das Verfahren ein Verfahren zum Beschichten.
Dabei ist die härtbaren Polymerzusammensetzung insbesondere eine Kunststoff-Beschichtung und wird insbesondere in einer Schichtdicke im Bereich von 0.5 bis 3 mm appliziert.

In einer Ausführungsform wird die Epoxidharz-Zusammensetzung als Klebstoff eingesetzt.
Auf Epoxidharzen basierende Klebstoffe werden typischerweise für Anwendungen eingesetzt, bei denen eine hohe Haftkraft, Festigkeit bzw. Steifigkeit und Bewitterungsstabilität gefordert ist, beispielsweise als Karrosserieklebstoff, Sandwichelementklebstoff, Brückenelementklebstoff oder Verankerungsklebstoff. Je nach Anwendung soll der Klebstoff bei Umgebungstemperatur verarbeitbar und schon früh belastbar sein und deshalb schnell Festigkeit aufbauen, insbesondere auch ohne zusätzlich erwärmt zu werden.

Ein weiterer Gegenstand der Erfindung ist somit ein Klebstoff, umfassend
- eine Harz-Komponente enthaltend mindestens ein Epoxidharz, wie vorgängig beschrieben, und
- eine Härter-Komponente enthaltend mindestens ein Reaktionsprodukt aus der Umsetzung von mindestens einem Amin der Formel (I) mit mindestens
einer Carbonylverbindung der Formel (II) und Wasserstoff, wie vorgängig beschrieben.

Bevorzugt enthält der Klebstoff zusätzlich mindestens einen weiteren Bestandteil ausgewählt aus Füllstoffen, Beschleunigern und weiteren Aminen, wie sie bereits genannt wurden.
Als Füllstoff geeignet sind insbesondere gemahlene oder gefällte Calciumcarbonate, welche gegebenenfalls mit Fettsäuren, insbesondere Stearaten, beschichtet sind, Quarzmehl, Quarzsand, Kaoline, Kieselsäuren, Zemente, Gipse oder Flugaschen.
Als Beschleuniger geeignet ist insbesondere Salicylsäure oder 2,4,6-Tris(dimethylaminomethyl)phenol oder eine Kombination davon.
Als weiteres Amin geeignet ist insbesondere N¹-Benzyl-1,2-propandiamin oder N²-Benzyl-1,2-propandiamin oder beliebige Gemische dieser Isomeren, N-Benzyl-1,3-bis(aminomethyl)benzol, N-Benzyl-1,3-bis(aminomethyl)cyclohexan, IPDA, MXDA, BHMT, TETA, TEPA, PEHA, DPTA, N4-Amin, DMAPA oder DMAPAPA.

Als Klebstoff weist die Zusammensetzung nach dem Vermischen der beiden Komponenten typischerweise eine dickflüssige bis pastöse Konsistenz auf. Bei der Applikation wird der frisch vermischte Klebstoff innerhalb seiner Offenzeit auf mindestens eines der zu verklebenden Substrate aufgetragen und die beiden Substrate innerhalb der Offenzeit des Klebstoffs zu einer Verklebung gefügt.
Als "Offenzeit" wird dabei die Zeitspanne zwischen dem Vermischen der Komponenten und dem Zeitpunkt, ab welchem keine ausreichende Verformbarkeit des Klebstoffs und/oder keine ausreichende Haftung zu den Substraten mehr gewährleistet ist.

Der frisch vermischte Klebstoff wird insbesondere mittels Pinsel, Rolle, Spatel, Rakel, Kelle, oder aus einer Tube, Kartusche oder Dosiervorrichtung appliziert bzw. aufgetragen.

Der Klebstoff ist besonders geeignet für das strukturelle Kleben in der Bau- oder Fertigungsindustrie, insbesondere als Klebemörtel, Montageklebstoff, Verankerungsklebstoff (Ankerklebstoff), Armierungsklebstoff wie insbesondere für das Verkleben von Lamellen aus CFK oder Stahl auf Beton, Mauerwerk oder Holz, als Elementklebstoff für beispielsweise Brücken, Sandwichelementklebstoff, Fassadenelementklebstoff, Verstärkungsklebstoff, Karrosserieklebstoff oder Halbschalenklebstoff für Rotorblätter.
Ebenfalls geeignet ist der Klebstoff für das Verfüllen von Hohlräumen wie Rissen, Spalten oder Bohrlöchern, wobei der Klebstoff in den Hohlraum gefüllt oder injiziert wird und nach der Aushärtung diesen ausfüllt und die Flanken des Hohlraums kraftschlüssig miteinander verbindet bzw. verklebt.

In einer Ausführungsform enthält der Klebstoff mindestens einen Füllstoff, insbesondere mindestens ein Quarzmehl oder mindestens einen Quarzsand. Ein solcher Klebstoff ist kostengünstig und besonders druckfest. Er ist insbesondere geeignet für Anwendungen im Baubereich, unter anderem als sogenannter Klebemörtel.

Aus der Verwendung des Klebstoffs entsteht ein verklebter Artikel. Der Artikel ist insbesondere ein Haus, ein Badezimmer, eine Küche, eine Treppe, ein Dach, ein Balkon, eine Terrasse, ein Parkdeck, eine Brücke, ein Tunnel, ein Sandwichelement einer Leichtbaustruktur, ein Solarpanel wie Photovoltaik- oder Solarthermie-Module, eine Fassade, ein Haushaltsapparat, ein Rotorblatt einer Windkraftanlage, ein Automobil, ein Bus, ein Lastkraftwagen, ein Schienenfahrzeug, ein Schiff, ein Flugzeug, ein Helikopter, oder ein Anbauteil eines solchen Artikels.

Der Klebstoff wird insbesondere eingesetzt in einem Verfahren zum Verkleben, umfassend die Schritte
- Mischen der Harz- und der Härter-Komponente mit einer geeigneten Methode, und
   entweder
   - Applizieren des vermischten Klebstoffs auf mindestens eine der zu verklebenden Substratoberflächen,
   - Fügen der Substrate zu einer Verklebung innerhalb der Offenzeit des Klebstoffs,
   oder
   - Applizieren des vermischten Klebstoffs in einen Hohlraum oder Spalt zwischen zwei Substraten,
   - gegebenenfalls Einfügen eines Ankers in den Hohlraum oder Spalt innerhalb der Offenzeit des Klebstoffs,
   gefolgt von der Aushärtung des Klebstoffs.

Als "Anker" wird dabei insbesondere ein Armierungseisen, ein Gewindestahl oder ein Bolzen bezeichnet. Ein solcher wird insbesondere so in einer Mauer, Wand, Decke oder in einem Boden eingeklebt bzw. verankert, dass ein Teil davon kraftschlüssig verklebt ist und ein Teil davon vorsteht und belastet werden kann.

Bevorzugt erfolgt die Applikation und die Aushärtung des Klebstoffs bei Umgebungstemperatur, insbesondere bei einer Temperatur im Bereich von 5 bis 35 °C, insbesondere 10 bis 30 °C. Dies ermöglicht eine besonders einfache Handhabung des Klebstoffs und ist insbesondere im Freien, auf Baustellen und in ungeheizten Industriehallen vorteilhaft.

Ein weiterer Gegenstand der Erfindung ist ein Reaktionsprodukt aus der Umsetzung von mindestens einem Amin der Formel (I) mit mindestens einer Carbonylverbindung der Formel (II) und Wasserstoff, wobei das Amin und die Carbonylverbindung in einem Molverhältnis im Bereich von 1/0.8 bis 1/1.1 in einem Lösemittel ohne Isolierung des als Zwischenprodukt gebildeten Imins und ohne Entfernung des freigesetzten Wassers mit molekularem Wasserstoff katalytisch hydriert werden, wobei das Reaktionsprodukt einen Gehalt an Amin der Formel (III) im Bereich von 30 bis 80 Gewichts-% aufweist.

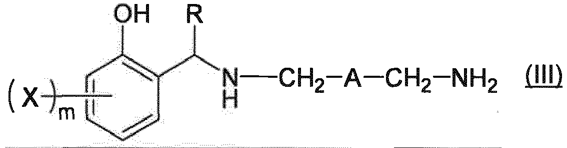

Bevorzugt wird bei einem Wasserstoff-Druck von 5 bis 100 bar, einer Temperatur von 40 bis 120 °C, insbesondere 60 bis 100 °C, und in Anwesenheit eines geeigneten Katalysators hydriert. Als Katalysator bevorzugt sind Palladium auf Kohle (Pd/C), Platin auf Kohle (Pt/C), Adams-Katalysator oder Raney-Nickel, insbesondere Palladium auf Kohle.

Bevorzugt werden das Amin und die Carbonylverbindung in einem Molverhältnis im Bereich von 1/0.9 bis 1/1 eingesetzt.

Als Lösemittel bevorzugt ist ein Alkohol oder ein Ester oder eine Mischung aus Alkohol und Ester. Besonders bevorzugt ist Ethanol oder Isopropanol oder Ethylacetat oder eine Kombination davon. Insbesondere geeignet ist Isopropanol oder eine Mischung aus Isopropanol und Ethylacetat.

Nach dem Hydrieren wird das Lösemittel bevorzugt destillativ entfernt, insbesondere zusammen mit dem freigesetzten Wasser.

Ein so hergestelltes Reaktionsprodukt ist besonders niedrigviskos und besonders reaktiv und somit ganz besonders geeignet für die Verwendung in einem Primer auf der Basis von Epoxidharz, welcher zur Vorbehandlung von porösen mineralischen Substraten geeignet ist.

Dadurch, dass das als Zwischenprodukt gebildete Imin vor der Hydrierung nicht isoliert wird und das freigesetzte Wasser nicht entfernt wird, weist das Reaktionsprodukt einen besonders hohen Gehalt an Amin der Formel (III) und einem besonders niedrigen Gehalt an mehrfach alkylierten Produkten auf, was bezüglich seiner verdünnenden Wirkung auf das Epoxidharz und seiner Reaktivität mit dem Epoxidharz äusserst vorteilhaft ist.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt. "AHEW" steht für das Aminwasserstoff-Equivalentgewicht.

"EEW" steht für das Epoxid-Equivalentgewicht. Als "Normklima" wird eine Temperatur von 23±1 °C und eine relative Luftfeuchtigkeit von 50±5% bezeichnet. "NK" steht für "Normklima".

### Beschreibung der Messmethoden:

**Infrarotspektren** (FT-IR) wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).
Die **Viskosität** wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm) bei einer Scherrate von 10 s⁻¹ gemessen.
Die **Aminzahl** wurde durch Titration bestimmt (mit 0.1N HClO₄ in Essigsäure gegen Kristallviolett).

### Verwendete kommerzielle Substanzen:

| | |
|---|---|
| Araldite® GY 250: | Bisphenol-A-Diglycidylether, EEW ca. 187.5 g/Eq (von Huntsman) |
| Araldite® DY-E: | Monoglycidylether von C₁₂- bis C₁₄-Alkoholen, EEW ca. 290 g/Eq (von Huntsman) |
| SR-Dur® 2750: | Mannich-Base auf Basis von 4-tert.Butylphenol, 1,3-Bis-(aminomethyl)benzol und Trimethyl-1,6-hexandiamin, enthaltend 10 bis 25 Gewichts-% 4-tert.Butylphenol, AHEW 75.0 g/Eq (von SRS Meeder) |

### Herstellung von erfindungsgemässen Reaktionsprodukten:

### Reaktionsprodukt A1: Reaktionsprodukt enthaltend N-(2-Hydroxybenzyl)-1,3-bis(aminomethyl)benzol

In einem Rundkolben wurden 27.24 g (0.20 mol) 1,3-Bis(aminomethyl)benzol (MXDA, von Mitsubishi Gas Chemicals) unter Stickstoffatmosphäre bei Raumtemperatur vorgelegt. Unter gutem Rühren wurde langsam eine Lösung von 24.42 g (0.20 mol) Salicylaldehyd in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche Flüssigkeit mit einer Viskosität von 22.1 Pa·s bei 20 °C und einer Aminzahl von 458 mg KOH/g.
FT-IR: 3022, 2846, 2721, 2613, 1587, 1454, 1254, 1082, 843, 747, 699.

### Reaktionsprodukt A2: Reaktionsprodukt enthaltend N-(2-Hydroxybenzyl)-1,3-bis(aminomethyl)cyclohexan

In einem Rundkolben wurden 14.22 g (0.10 mol) 1,3-Bis(aminomethyl)cyclohexan (1,3-BAC, von Mitsubishi Gas Chemicals) unter Stickstoffatmosphäre bei Raumtemperatur vorgelegt. Unter gutem Rühren wurde langsam eine Lösung von 12.21 g (0.10 mol) Salicylaldehyd in 350 ml Isopropanol dazugetropft und 2 Stunden nachgerührt. Danach wurde die Reaktionsmischung bei einem Wasserstoff-Druck von 90 bar, einer Temperatur von 90 °C und einem Fluss von 4 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche Flüssigkeit mit einer Viskosität von 124 Pa·s bei 20 °C und einer Aminzahl von 445 mg KOH/g.
FT-IR: 2915, 2846, 2726, 1589, 1455, 1413, 1257, 1151, 1102, 1036, 953, 931, 842, 793, 720.

### Herstellung von nicht erfindungsgemässen Reaktionsprodukten als Vergleich:

### Reaktionsprodukt R1: Reaktionsprodukt enthaltend N-(2-Hydroxybenzyl)-4-methyl-1,5-pentandiamin

In einem Rundkolben wurden 23.24 g (0.20 mol) 1,5-Diamino-2-methylpentan (Dytek® A, von Invista) unter Stickstoffatmosphäre bei Raumtemperatur vorgelegt. Unter gutem Rühren wurde langsam eine Lösung von 24.42 g (0.20 mol) Salicylaldehyd in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche Flüssigkeit mit einer Viskosität von 2.3 Pa·s bei 20 °C und einer Aminzahl von 506 mg KOH/g.

### Reaktionsprodukt R2: Reaktionsprodukt enthaltend N-(2-Hydroxybenzyl)-3,3(5),5-trimethyl-1,6-hexandiamin

In einem Rundkolben wurden 15.82 g (0.10 mol) 2,2(4),4-Trimethylhexamethylendiamin (Vestamin® TMD, von Evonik) unter Stickstoffatmosphäre bei Raumtemperatur vorgelegt. Unter gutem Rühren wurde langsam eine Lösung von 12.21 g (0.10 mol) Salicylaldehyd in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 4 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine dunkelgelbe Flüssigkeit mit einer Viskosität von 4.4 Pa·s bei 20 °C und einer Aminzahl von 414 mg KOH/g.

### Reaktionsprodukt R3: Reaktionsprodukt enthaltend N-(2-Hydroxybenzyl)-4,7-diaza-1,10-decandiamin

In einem Rundkolben wurden 34.85 g (0.20 mol) N,N'-Bis(3-aminopropyl)ethylendiamin (N4-Amine, von BASF) unter Stickstoffatmosphäre bei Raumtemperatur vorgelegt. Unter gutem Rühren wurde langsam eine Lösung von 24.42 g (0.20 mol) Salicylaldehyd in 400 ml Isopropanol dazugetropft und 2 Stunden nachgerührt. Danach wurde die Reaktionsmischung bei einem Wasserstoff-Druck von 85 bar, einer Temperatur von 90 °C und einem Fluss von 4 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche Flüssigkeit mit einer Viskosität von 2.5 Pa·s bei 20 °C und einer Aminzahl von 794 mg KOH/g.

### Reaktionsprodukt R4: Reaktionsprodukt enthaltend N-Benzyl-1,3-bis(aminomethyl)benzol

In einem Rundkolben wurden 27.24 g (0.20 mol) 1,3-Bis(aminomethyl)benzol (MXDA, von Mitsubishi Gas Chemicals) unter Stickstoffatmosphäre bei Raumtemperatur vorgelegt. Unter gutem Rühren wurde langsam eine Lösung von 26.53 g (0.25 mol) Benzaldehyd in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche Flüssigkeit mit einer Viskosität von 0.1 Pa·s bei 20 °C und einer Aminzahl von 438 mg KOH/g.

### Reaktionsprodukt R5: Reaktionsprodukt enthaltend N-(4-Hydroxybenzyl)-1,3-bis(aminomethyl)benzol

In einem Rundkolben wurden 27.24 g (0.20 mol) 1,3-Bis(aminomethyl)benzol (MXDA, von Mitsubishi Gas Chemicals) unter Stickstoffatmosphäre bei Raumtemperatur vorgelegt. Unter gutem Rühren wurde langsam eine Lösung von 24.42 g (0.20 mol) 4-Hydroxybenzaldehyd in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche hochviskose Flüssigkeit mit einer Viskosität von > 200 Pa·s bei 20 °C und 8 Pa·s bei 60 °C.

### Reaktionsprodukt R6: Reaktionsprodukt enthaltend N-(4-Hydroxy-2-methoxybenzyl)-1,3-bis(aminomethyl)benzol

In einem Rundkolben wurden 27.24 g (0.20 mol) 1,3-Bis(aminomethyl)benzol (MXDA, von Mitsubishi Gas Chemicals) unter Stickstoffatmosphäre bei Raumtemperatur vorgelegt. Unter gutem Rühren wurde langsam eine Lösung von 30.43 g (0.20 mol) Vanillin in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine orangefarbene hochviskose Flüssigkeit mit einer Viskosität von > 200 Pa·s bei 20 °C und 15 Pa·s bei 60 °C.

### Reaktionsprodukt R7: N,N'-Bis(2-hydroxybenzyl)-1,3-bis(aminomethyl)benzol

In einem Rundkolben wurden 13.62 g (0.10 mol) 1,3-Bis(aminomethyl)benzol (MXDA, von Mitsubishi Gas Chemicals), 24.42 g (0.20 mol) Salicylaldehyd und 400 ml Isopropanol unter Stickstoffatmosphäre vermischt und 2 Stunden bei Raumtemperatur gerührt. Danach wurde die Reaktionsmischung bei einem Wasserstoff-Druck von 85 bar, einer Temperatur von 90 °C und einem Fluss von 4 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche hochviskose Flüssigkeit mit einer Viskosität von > 200 Pa·s bei 20 °C und 2.9 Pa·s bei 60 °C und einer Aminzahl von 303 mg KOH/g.

### Herstellung von Epoxidharz-Zusammensetzungen:

### Beispiele 1 bis 7

Für jedes Beispiel wurden die in der Tabelle 1 angegebenen Inhaltsstoffe in den angegebenen Mengen (in Gewichtsteilen) der Harz-Komponente mittels eines Zentrifugalmischers (SpeedMixer™ DAC 150, FlackTek Inc.) vermischt und unter Ausschluss von Feuchtigkeit aufbewahrt.
Anschliessend wurde das in der Tabelle 1 angegebene Reaktionsprodukt in der angegebenen Menge mittels des Zentrifugalmischers mit der Harz-Komponente vermischt, zu einer homogenen Flüssigkeit verarbeitet und diese unverzüglich folgendermassen eingesetzt und geprüft:
5 Minuten nach dem Vermischen wurde die Viskosität bei 20°C bestimmt ("**Viskosität (5')**").
Ein erster Film wurde in einer Schichtdicke von 500 µm auf eine Glasplatte aufgezogen und dieser im Normklima gelagert bzw. ausgehärtet. An diesem Film wurde die **Königshärte** (Pendelhärte nach König gemäss DIN EN ISO 1522, "Königshärte (NK)") nach 1 Tag (1d), 2 Tagen (2d), 4 Tagen (4d) und 7 Tagen (7d) bestimmt. Weiterhin wurde der Aspekt des ausgehärteten Films beurteilt (in der Tabelle 1 mit "Aspekt (NK)" bezeichnet). Als "schön" wurde dabei ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet.
Ein zweiter Film wurde in einer Schichtdicke von 500 µm auf eine Glasplatte aufgezogen und dieser unmittelbar nach dem Applizieren während 7 Tagen bei 8 °C und 80 % relativer Feuchtigkeit und anschliessend im Normklima gelagert bzw. ausgehärtet. Dabei wurde ebenfalls die Königshärte bestimmt, jeweils nach 1 Tag (1d 8°/80%), 2 Tagen (2d 8°/80%), 7 Tagen (7d 8°/80%) und 8 Tagen (+1d NK). Anschliessend wurde der Aspekt dieses Films beurteilt (in der Tabelle 1 mit "Aspekt (8°/80%)" bezeichnet), auf die gleiche Weise wie für den Aspekt (NK) beschrieben.
Als Mass für die Vergilbung wurde weiterhin die Farbveränderung nach Belastung in einem Bewitterungstester bestimmt. Dazu wurde ein weiterer Film in einer Schichtdicke von 500 µm auf eine Glasplatte aufgezogen und dieser im Normklima während 2 Wochen gelagert bzw. ausgehärtet und anschliessend in einem Bewitterungstester des Typs Q-Sun Xenon Xe-1 mit optischem Filter Q-SUN Daylight-Q und einer Xenon Lampe mit einer Lichtstärke von 0.51 W/m² bei 340 nm bei einer Temperatur von 65°C während 72 Stunden belastet (**Q-Sun (72h)**). Anschliessend wurde der Farbunterschied ΔE des so belasteten Films im Vergleich zum entsprechenden nicht belasteten Film mittels einem Colorimeter NH310 von Shenzen 3NH Technology Co. LTD, ausgerüstet mit Silicon Photoelectric Diode Detector, Light Source A, Color Space Measurement Interface CIE L*a*b*C*H*, bestimmt. Ein hoher ΔE-Wert steht dabei für eine grossen Farbunterschied bzw. eine starke Vergilbung.
Die Resultate sind in der Tabelle 1 angegeben.

Diese Epoxidharz-Zusammensetzungen sind geeignet als Primer oder als Klebstoff. Die Viskosität nach dem Vermischen der beiden Komponenten ist dabei ein Mass für die Verarbeitbarkeit als Primer. Die Entwicklung der Königshärte ist dabei ein Mass für die Aushärtungsgeschwindigkeit und die Endhärte im jeweiligen Klima.

Die mit "(Ref.)" bezeichneten Beispiele sind Vergleichsbeispiele.

**Tabelle 1: Zusammensetzung und Eigenschaften der Beispiele 1 bis 7.**

| **Beispiel** | | **1** | **2** | **3 (Ref.)** | **4 (Ref.)** | **5 (Ref.)** | **6 (Ref.)** | **7 (Ref.)** |
|---|---|---|---|---|---|---|---|---|
| **Harz-Komp.:** | | | | | | | | |
| Araldite® GY-250 | | 167.2 | 167.2 | 167.2 | 167.2 | 167.2 | 167.2 | 167.2 |
| Araldite® DY-E | | 31.8 | 31.8 | 31.8 | 31.8 | 31.8 | 31.8 | 31.8 |

| **Härter-Komp.:** | | | | | | | | |
|---|---|---|---|---|---|---|---|---|
| Reaktionsprodukt | | **A1** | **A2** | | **R1** | **R2** | **R3** | **R4** |
| | | 81.0 | 82.8 | - | 74.1 | 88.1 | 56.1 | 95.0 |
| SR-Dur® 2750 | | - | - | 75.0 | - | - | - | - |
| Viskosität (5') [Pa·s] | | 3.5 | 5.1 | 3.3 | 1.7 | 2.4 | 2.6 | 0.51 |
| Königshärte [s] | (1d NK) | 165 | 171 | 168 | 60 | 31 | 74 | 56 |
| | (2d NK) | 197 | 198 | 182 | 108 | 71 | 87 | 140 |
| | (4d NK) | 202 | 204 | 203 | 138 | 110 | 87 | 183 |
| | (7d NK) | 207 | 210 | 205 | 155 | 153 | 95 | 204 |
| Aspekt (NK) | | leicht matt | schön | schön | leichte Struktur | schön | klebrig, matt | leicht matt |
| Q-Sun (72h) ΔE | | 5.8 | 5.9 | 23.0 | 12.3 | 5.7 | 6.1 | n.b. |
| Königsh. [s] | (1d 8°/80%) | 14 | 24 | 27 | n.b. | n.b. | n.b. | n.b. |
| | (2d 8°/80%) | 74 | 83 | 70 | n.b. | n.b. | n.b. | n.b. |
| | (7d 8°/80%) | 140 | 155 | 120 | 27 | 33 | 52 | 63 |
| | (+1d NK) | 190 | 182 | 148 | 42 | 84 | 92 | 121 |
| Aspekt (8°/80%) | | leicht matt | matt, leichte Struktur | leichte Struktur | matt, leichte Struktur | leicht matt | trüb, klebrig, Struktur | trüb, Struktur |

| | | | | | | | | |
|---|---|---|---|---|---|---|---|---|
| "n.b." steht für "nicht bestimmt" | | | | | | | | |

### Herstellung von Primern:

### Beispiele 8 bis 18

Für jedes Beispiel wurden die in den Tabellen 2 bis 3 angegebenen Inhaltsstoffe in den angegebenen Mengen (in Gewichtsteilen) der Härter-Komponente mittels eines Zentrifugalmischers (SpeedMixer™ DAC 150, FlackTek Inc.) vermischt und unter Ausschluss von Feuchtigkeit aufbewahrt.
Ebenso wurden die in den Tabellen 2 bis 3 angegebenen Inhaltsstoffe der Harz-Komponente verarbeitet und aufbewahrt.

Anschliessend wurden die beiden Komponenten jedes Primers bei Raumtemperatur mittels des Zentrifugalmischers zu einer homogenen Flüssigkeit verarbeitet und diese unverzüglich folgendermassen eingesetzt und geprüft:
5 Minuten nach dem Vermischen wurde die Viskosität bei 20°C bestimmt ("**Viskosität (5')**").
Jeder Primer wurde auf drei Gartenplatten aus Beton mittels eines Pinsels so aufgetragen, dass deren Oberfläche durch den Primer vollständig bedeckt war und die Poren durch den Primer verschlossen wurden. Anschliessend wurden eine Gartenplatte im Normklima und zwei Gartenplatten bei 8°C und 80 % relativer Luftfeuchtigkeit aufbewahrt. Zur Bestimmung der Zeit bis zur **Begehbarkeit** wurde darauf im Normklima (NK) bzw. bei 8°C und 80 % relativer Luftfeuchtigkeit (8°/80%) die Zeit bestimmt, die es brauchte, bis ein während 60 Sekunden auf der Oberfläche stehender Metallzylinder von 24 mm Durchmesser und einem Gewicht von 1 kg nicht mehr anklebte und keine Markierung hinterliess.
Die Resultate sind in den Tabellen 2 bis 3 angegeben.

Die mit "(Ref.)" bezeichneten Beispiele sind nicht erfindungsgemäss und dienen als Vergleich.

**Tabelle 2: Zusammensetzung (in Gewichtsteilen) und Eigenschaften der Primer der Beispiele 8 bis 16. "n.b." steht für "nicht bestimmt" ¹ zu hochviskos**

| **Beispiel** | | **8** | **9** | **10 (Ref.)** | **11 (Ref.)** | **12 (Ref.)** | **13 (Ref.)** | **14 (Ref.)** | **15 (Ref.)** | **16 (Ref.)** |
|---|---|---|---|---|---|---|---|---|---|---|
| **Harz-Komponente:** | | | | | | | | | | |
| Araldite® GY-250 | | 167.2 | 167.2 | 167.2 | 167.2 | 167.2 | 167.2 | 167.2 | 167.2 | 167.2 |
| Araldite® DY-E | | 31.8 | 31.8 | 31.8 | 31.8 | 31.8 | 31.8 | 31.8 | 31.8 | 31.8 |

| **Härter-Komponente:** | | | | | | | | | | |
|---|---|---|---|---|---|---|---|---|---|---|
| Reaktionsprodukt | | **A1** | **A2** | - | **R1** | **R2** | **R4** | **R5** | **R6** | **R7** |
| | | 81.0 | 82.8 | | 74.1 | 88.1 | 95.0 | 81.0 | 90.8 | 174.0 |
| SR-Dur® 2750 | | - | - | 75.0 | - | - | | - | - | - |
| Benzylalkohol | | 34.0 | 35.5 | 32.0 | 31.8 | 31.8 | 34.0 | 34.0 | 34.0 | 40.0 |
| Viskosität (5') [Pa·s] | | 1.5 | 2.5 | 2.2 | 1.1 | 1.3 | 0.41 | 8.1¹ | 12.3¹ | 5.1 |
| Begehbarkeit | | | | | | | | | | |
| | im NK | 6.5h | 6h | 4.5h | 8h | 8h | 12h | n.b. | n.b. | 12h |
| | bei 8°/80% | 22h | 20h | 22h | 30h | 48h | 48h | n.b. | n.b. | 55h |

**Tabelle 3: Zusammensetzung (in Gewichtsteilen) und Eigenschaften der Primer der Beispiele 17 und 18.**

| **Beispiel** | **17** | **18 (Ref.)** |
|---|---|---|
| **Harz-Komponente:** | | |
| Araldite® GY-250 | 167.2 | 167.2 |
| Araldite® DY-E | 31.8 | 31.8 |

| **Härter-Komponente:** | | |
|---|---|---|
| Reaktionsprodukt | **A1** | - |
| | 81.0 | |
| SR-Dur® 2750 | - | 75.0 |
| Cardanol¹ | 34.6 | 32.0 |
| Viskosität (5') [Pa·s] | 3.8 | 4.9 |
| Begehbarkeit | | |
| im NK | 5h | 4h |
| bei 8°/80% | 18h | 15h |

| | | |
|---|---|---|
| ¹Cardolite® NX-2026 von Cardolite Corp. | | |

### Herstellung von Beschichtungen:

### Beispiele 19 bis 24

Die mit erfindungemässen Primern grundierten Gartenplatten der Beispiele **8, 9** und **17** wurden nach 24 Stunden Aushärtung bei 8°C und 80% relativer Luftfeuchtigkeit jeweils mit einer kommerziellen Bodenbeschichtung beschichtet, wie in Tabelle 4 angegeben. Dabei wurde entweder Sikafloor®-264 kieselgrau (2-komponentige Epoxidharz-Beschichtung, von Sika Schweiz AG) in einer Schichtdicke von 1.5 mm aufgetragen, indem die frisch vermischte Beschichtung auf die mit dem jeweiligen Primer grundierte Gartenplatte aufgegossen und mit einer Rakel verteilt wurde. Oder es wurde Sikafloor®-400 N Elastic kieselgrau (flexible 1-komponentige Polyurethan-Beschichtung, von Sika Schweiz AG) in einer Schichtdicke von 1.5 mm aufgetragen, indem diese auf die mit dem jeweiligen Primer grundierte Gartenplatte aufgegossen und mit einer Rakel verteilt wurde.

Nach einer Aushärtungszeit von 7 Tagen im Normklima war auf jeder Gartenplatte ein qualitativ hochwertiger, mehrschichtiger Bodenbelag mit fehlerfreier Oberfläche und hervorragender Haftung zwischen den Schichten vorhanden.

**Tabelle 4: Aufbau der Beschichtungen der Beispiele 19 bis 24.**

| **Beispiel** | **19** | **20** | **21** | **22** | **23** | **24** |
|---|---|---|---|---|---|---|
| Primer aus Beispiel | 8 | 8 | 9 | 9 | 17 | 17 |
| Sikafloor® Typ | 264 | 400 N | 264 | 400 N | 264 | 400 N |

### Herstellung von Klebstoffen:

### Beispiele 25 und 26

Für das Beispiel **25** wurden die in der Tabelle 5 angegebenen Inhaltsstoffe in den angegebenen Mengen (in Gewichtsteilen) der Härter-Komponente mittels eines Zentrifugalmischers (SpeedMixer™ DAC 150, FlackTek Inc.) vermischt und unter Ausschluss von Feuchtigkeit aufbewahrt.
Für das Beispiel **26** (Vergleich) wurde als Härter-Komponente Sikadur® 31 CF Normal Komponente B (von Sika Schweiz AG) in der in Tabelle 5 angegeben Menge verwendet.

Als Harz-Komponente wurde jeweils Sikadur® 31 CF Normal Komponente A (von Sika Schweiz AG) mit EEW ca. 838 g in der in Tabelle 5 angegeben Menge verwendet.
Anschliessend wurden die beiden Komponenten bei Raumtemperatur mittels des Zentrifugalmischers zu einer homogenen Paste verarbeitet und diese unverzüglich folgendermassen eingesetzt und geprüft:
Es wurden mehrere Verklebungen hergestellt, indem jeweils einige Gramm Klebstoff auf eine mittels Stahlbürste gereinigte Betonplatte aufgetragen und darauf ein Zylinder aus mittels Aceton gereinigtem Stahl mit einem Durchmesser von 20 mm über dessen Grundfläche aufgeklebt wurde, wobei die Dicke der Klebefuge 2 mm betrug. Die Verklebungen wurden im Normklima gelagert. Nach 2 Tagen wurden sie in Anlehnung an DIN EN 4624 bei einer Prüfgeschwindigkeit von 2 mm/min bis zum Bruch auseinandergezogen, um die Festigkeit der Verklebung bei der maximalen Kraft zu bestimmen (**Haftzug Beton-Stahl**).
Weiterhin wurden mehrere Verklebungen hergestellt, indem jeweils zwei Zylinder aus mittels Aceton gereinigtem Stahl mit einem Durchmesser von 20 mm mit dem Klebstoff auf der runden Grundfläche so zusammenklebt wurden, dass die Dicke der Klebefuge 2 mm betrug. Die Verklebungen wurden im Normklima gelagert. Nach 2 Tagen wurden sie in Anlehnung an DIN EN ISO 4624 bei einer Prüfgeschwindigkeit von 2 mm/min bis zum Bruch auseinandergezogen, um die Festigkeit der Verklebung bei der maximalen Kraft zu bestimmen (**Haftzug Stahl-Stahl**).
Weiterhin wurden die **Druckfestigkeit** bestimmt, indem der Klebstoff in Form von Quadern der Dimensionen 12.7 x 12.7 x 25.4 mm im Normklima ausgehärtet und aufbewahrt wurde. Nach 2 und nach 7 Tagen wurden jeweils mehrere solche Quader nach ASTM D695 mit einer Prüfgeschwindigkeit von 1.3 mm/min bis zur Zerstörung zusammengedrückt, wobei der Wert für die Druckfestigkeit jeweils bei der maximalen Kraft abgelesen wurde.
Die Resultate sind in der Tabelle 5 angegeben.

Das mit "(Ref.)" bezeichnete Beispiel 26 ist nicht erfindungsgemäss und dient als Vergleich.

**Tabelle 5: Zusammensetzung (in Gewichtsteilen) und Eigenschaften der Klebstoffe der Beispiele 25 und 26.**

| **Beispiel** | | **25** | **26 (Ref.)** |
|---|---|---|---|
| **Harz-Komponente:** | | | |
| Sikadur® 31 Komponente A | | 200.0 | 200.0 |

| **Härter-Komponente:** | | | |
|---|---|---|---|
| Sikadur® 31 Komponente B | | - | 100 |
| Reaktionsprodukt **A1** | | 21.0 | - |
| Eisenoxid Pigment schwarz | | 0.1 | - |
| Quarzmehl 0-75 µm | | 36.0 | - |
| Quarzsand 0.1-0.3 mm | | 27.9 | - |
| gefällte, mit Stearat beschichtete Kreide | | 15.0 | - |
| Haftzug Beton-Stahl | 2 Tage NK | 3.7 MPa¹ | 4.4 MPa¹ |
| Haftzug Stahl-Stahl | 2 Tage NK | 15.2 MPa | 24.5 MPa |
| Druckfestigkeit | 2 Tage NK | 77.6 MPa | 56.2 MPa |
| | 7 Tage NK | 86.8 MPa | 64.9 MPa |

| | | | |
|---|---|---|---|
| ¹ Bruch im Beton | | | |

## Patentansprüche

1. Verwendung einer Epoxidharz-Zusammensetzung enthaltend mindestens ein Reaktionsprodukt aus der Umsetzung von mindestens einem Amin der Formel (I) mit mindestens einer Carbonylverbindung der Formel (II) und Wasserstoff,
NH₂-CH₂-A-CH₂-NH₂ (I) 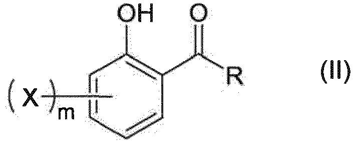
wobei
A für einen Phenylen-Rest oder Cyclohexylen-Rest steht,
R für einen Wasserstoff-Rest oder Alkyl-Rest mit 1 bis 8 C-Atomen steht,
m für 0 oder 1 steht, und
X für einen Hydroxyl-Rest oder Methyl-Rest oder Methoxy-Rest steht,
wobei das Molverhältnis zwischen dem Amin der Formel (I) und der Carbonylverbindung der Formel (II) im Bereich von 1/0.7 bis 1/1.2 liegt,
als kalthärtender Primer oder Klebstoff,
**dadurch gekennzeichnet, dass** das Reaktionsprodukt einen Gehalt an Amin der Formel (III) im Bereich von 30 bis 80 Gewichts-% aufweist.

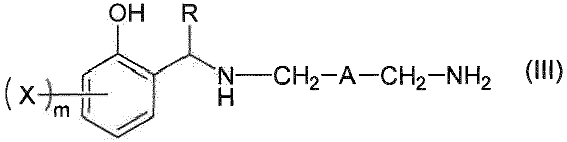

2. Verwendung gemäss Anspruch 1, **dadurch gekennzeichnet, dass** das Amin der Formel (I) ausgewählt ist aus der Gruppe bestehend aus 1,3-Bis(aminomethyl)benzol und 1,3-Bis(aminomethyl)cyclohexan.

3. Verwendung gemäss einem der Ansprüche 1 oder 2, **dadurch gekennzeichnet, dass** die Carbonylverbindung der Formel (II) ausgewählt ist aus der Gruppe bestehend aus Salicylaldehyd und 2'-Hydroxyacetophenon.

4. Primer, insbesondere zur Vorbehandlung von porösen mineralischen Substraten, umfassend
- eine Harz-Komponente enthaltend mindestens ein Epoxidharz und
- eine Härter-Komponente enthaltend mindestens ein Reaktionsprodukt wie in einem der Ansprüche 1 bis 3 beschrieben.

5. Primer gemäss Anspruch 4, **dadurch gekennzeichnet, dass** er eine Viskosität bei 20 °C von höchstens 5 Pa·s aufweist, bestimmt wie in der Beschreibung angegeben.

6. Primer gemäss einem der Ansprüche 4 oder 5, **dadurch gekennzeichnet, dass** er mindestens einen Verdünner ausgewählt aus der Gruppe bestehend aus Benzylalkohol, styrolisiertem Phenol, ethoxyliertem Phenol, phenolgruppenhaltigen aromatische Kohlenwasserstoffharzen und Cardanol enthält.

7. Primer gemäss einem der Ansprüche 4 bis 6, **dadurch gekennzeichnet, dass** die Härter-Komponente weniger als 0.1 Gewichts-% Phenol-Verbindungen ausgewählt aus der Gruppe bestehend aus Phenol, Kresol, Resorcin, tert.Butylphenol, Nonylphenol und Dodecylphenol enthält.

8. Klebstoff, umfassend
- eine Harz-Komponente enthaltend mindestens ein Epoxidharz und
- eine Härter-Komponente enthaltend mindestens ein Reaktionsprodukt wie in einem der Ansprüche 1 bis 3 beschrieben.

9. Schicht-Aufbau, umfassend
- mindestens eine Schicht des Primers gemäss einem der Ansprüche 4 bis 7,
- mindestens eine Schicht einer härtbaren Polymerzusammensetzung,
- gegebenenfalls eine Deckschicht und/oder Versiegelung.

10. Schicht-Aufbau gemäss Anspruch 9, **dadurch gekennzeichnet, dass** dieser ein Beschichtungs-System darstellt, wobei die härtbare Polymerzusammensetzung eine Kunststoff-Beschichtung ist.

11. Schicht-Aufbau gemäss Anspruch 10, **dadurch gekennzeichnet, dass** dieser einen Boden oder eine Schutzbeschichtung oder eine Wasserabdichtung darstellt.

12. Verfahren zum Beschichten oder Verkleben eines porösen mineralischen Substrats, umfassend die Schritte
(i) Applizieren des Primers gemäss einem der Ansprüche 4 bis 7 auf das poröse mineralische Substrat, insbesondere in einer zum Verschliessen der Poren ausreichenden Menge,
(ii) Aushärten des applizierten Primers bei Umgebungstemperatur, insbesondere mindestens bis zur Begehbarkeit,
(iii) Applizieren von mindestens einer Schicht einer härtbaren Polymerzusammensetzung auf den applizierten und zumindest teilweise ausgehärteten Primer.

13. Verfahren gemäss Anspruch 12, **dadurch gekennzeichnet, dass** der Primer bei einer Temperatur unterhalb von 20 °C appliziert und/oder ausgehärtet wird.

14. Reaktionsprodukt aus der Umsetzung von mindestens einem Amin der Formel (I) mit mindestens einer Carbonylverbindung der Formel (II) und Wasserstoff,
NH₂-CH₂-A-CH₂-NH₂ (I) 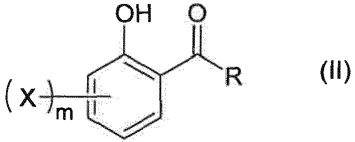
wobei
A für einen Phenylen-Rest oder Cyclohexylen-Rest steht,
R für einen Wasserstoff-Rest oder Alkyl-Rest mit 1 bis 8 C-Atomen steht,
m für 0 oder 1 steht, und
X für einen Hydroxyl-Rest oder Methyl-Rest oder Methoxy-Rest steht,
wobei das Amin und die Carbonylverbindung in einem Molverhältnis im Bereich von 1/0.8 bis 1/1.1 in einem Lösemittel ohne Isolierung des als Zwischenprodukt gebildeten Imins und ohne Entfernung des freigesetzten Wassers mit molekularem Wasserstoff katalytisch hydriert werden, **dadurch gekennzeichnet, dass** das Reaktionsprodukt einen Gehalt an Amin der Formel (III) im Bereich von 30 bis 80 Gewichts-% aufweist.

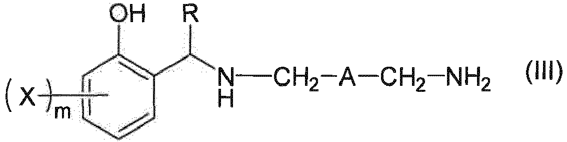

## Claims

1. Use of an epoxy resin composition comprising at least one reaction product from the reaction of at least one amine of the formula (I) with at least one carbonyl compound of the formula (II) and hydrogen,
NH₂-CH₂-A-CH₂-NH₂ (I) 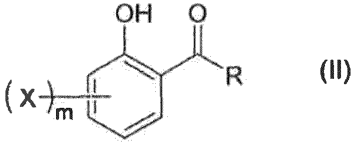
where
A is a phenylene radical or cyclohexylene radical,
R is a hydrogen radical or alkyl radical having 1 to 8 carbon atoms,
m is 0 or 1, and
X is a hydroxyl radical or methyl radical or methoxy radical,
where the molar ratio between the amine of the formula (I) and the carbonyl compound of the formula (II) is in the range from 1/0.7 to 1/1.2, as a cold-curing primer or adhesive,
**characterized in that** the reaction product contains an amount of amine of the formula (III) in the range from 30 to 80 weight%.

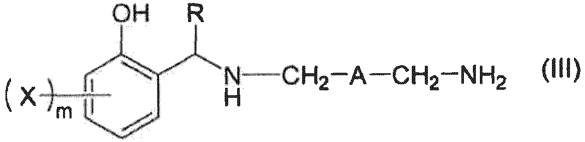

2. Use according to Claim 1, **characterized in that** the amine of the formula (I) is selected from the group consisting of 1,3-bis(aminomethyl)benzene and 1,3-bis(aminomethyl)cyclohexane.

3. Use according to either of Claims 1 and 2, **characterized in that** the carbonyl compound of the formula (II) is selected from the group consisting of salicylaldehyde and 2'-hydroxyacetophenone.

4. Primer, more particularly for pretreating porous mineral substrates, comprising
- a resin component comprising at least one epoxy resin and
- a hardener component comprising at least one reaction product as described in any of Claims 1 to 3.

5. Primer according to Claim 4, **characterized in that** it has a viscosity at 20°C of not more than 5 Pa·s, determined as specified in the description.

6. Primer according to either of Claims 4 and 5, **characterized in that** it comprises at least one diluent selected from the group consisting of benzyl alcohol, styrenized phenol, ethoxylated phenol, aromatic hydrocarbon resins containing phenol groups, and cardanol.

7. Primer according to any of Claims 4 to 6, **characterized in that** the hardener component comprises less than 0.1 weight% of phenol compounds selected from the group consisting of phenol, cresol, resorcinol, tert-butylphenol, nonylphenol and dodecylphenol.

8. Adhesive comprising
- a resin component comprising at least one epoxy resin and
- a hardener component comprising at least one reaction product as described in any of Claims 1 to 3.

9. Layer structure comprising
- at least one layer of the primer according to any of Claims 4 to 7,
- at least one layer of a curable polymer composition,
- optionally an outer layer and/or sealing.

10. Layer structure according to Claim 9, **characterized in that** it represents a coating system, where the curable polymer composition is a plastics coating.

11. Layer structure according to Claim 10, **characterized in that** it represents a floor or a protective coating or a water seal.

12. Method for coating or bonding a porous mineral substrate, comprising the steps of
(i) applying the primer according to any of Claims 4 to 7 to the porous mineral substrate, more particularly in an amount sufficient to close the pores,
(ii) curing the applied primer at ambient temperature, in particular at least to capacity to accept foot traffic,
(iii) applying at least one layer of a curable polymer composition to the applied and at least partly cured primer.

13. Method according to Claim 12, **characterized in that** the primer is applied and/or cured at a temperature below 20°C.

14. Reaction product from the reaction of at least one amine of the formula (I) with at least one carbonyl compound of the formula (II) and hydrogen,
NH₂-CH₂-A-CH₂-NH₂ (I) 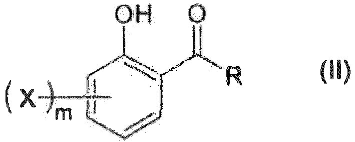
where
A is a phenylene radical or cyclohexylene radical,
R is a hydrogen radical or alkyl radical having 1 to 8 carbon atoms,
m is 0 or 1, and
X is a hydroxyl radical or methyl radical or methoxy radical,
where the amine and the carbonyl compound in a molar ratio in the range from 1/0.8 to 1/1.1 are subjected to catalytic hydrogenation with molecular hydrogen in a solvent, without isolation of the imine intermediate form and without removal of the water liberated,
**characterized in that** the reaction product contains an amount of amine of the formula (III) in the range from 30 to 80 weight%.

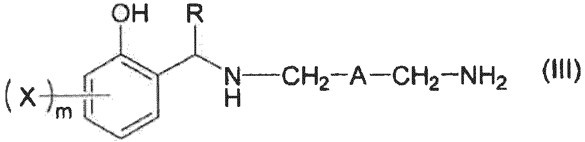

## Revendications

1. Utilisation d'une composition de résine époxyde contenant au moins un produit de réaction issu de la mise en réaction d'au moins une amine de la formule (I) avec au moins un composé de carbonyle de la formule (II) et de l'hydrogène,
NH₂-CH₂-A-CH₂-NH₂ (I) 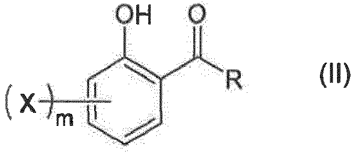
dans lesquelles
A représente un radical phénylène ou un radical cyclohexylène,
R représente un radical hydrogène ou un radical alkyle de 1 à 8 atomes C,
m représente 0 ou 1, et
X représente un radical hydroxyle ou un radical méthyle ou un radical méthoxy,
le rapport en moles entre l'amine de la formule (I) et le composé de carbonyle de la formule (II) se situant dans la plage allant de 1/0,7 à 1/1,2,
en tant qu'amorce ou adhésif durcissant au froid,
**caractérisée en ce que** le produit de réaction présente une teneur en amine de la formule (III) dans la plage allant de 30 à 80 % en poids

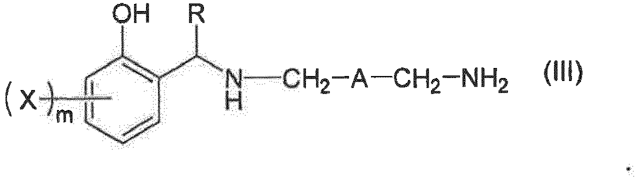

2. Utilisation selon la revendication 1, **caractérisée en ce que** l'amine de la formule (I) est choisie dans le groupe constitué par le 1,3-bis (aminométhyl) benzène et le 1,3-bis(aminométhyl)cyclohexane.

3. Utilisation selon l'une quelconque des revendications 1 ou 2, **caractérisée en ce que** le composé de carbonyle de la formule (II) est choisi dans le groupe constitué par le salicylaldéhyde et la 2'-hydroxyacétophénone.

4. Amorce, notament pour le prétraitement de substrats minéraux poreux, comprenant :
- un composant résine contenant au moins une résine époxyde et
- un composant durcisseur contenant au moins un produit de réaction tel que décrit dans l'une quelconque des revendications 1 à 3.

5. Amorce selon la revendication 4, **caractérisée en ce qu'**elle présente une viscosité à 20 °C d'au plus 5 Pa·s, déterminée tel qu'indiqué dans la description.

6. Amorce selon l'une quelconque des revendications 4 ou 5, **caractérisée en ce qu'**elle contient au moins un diluant choisi dans le groupe constitué par l'alcool benzylique, le phénol styrénisé, le phénol éthoxylé, les résines hydrocarbonées aromatiques contenant des groupes phénol et le cardanol.

7. Amorce selon l'une quelconque des revendications 4 à 6, **caractérisée en ce que** le composant durcisseur contient moins de 0,1 % en poids de composés de phénol choisis dans le groupe constitué par le phénol, le crésol, la résorcine, le tert.-butylphénol, le nonylphénol et le dodécylphénol.

8. Adhésif, comprenant :
- un composant résine contenant au moins une résine époxyde et
- un composant durcisseur contenant au moins un produit de réaction tel que décrit dans l'une quelconque des revendications 1 à 3.

9. Structure de couches, comprenant :
- au moins une couche de l'amorce selon l'une quelconque des revendications 4 à 7,
- au moins une couche d'une composition de polymère durcissable,
- éventuellement une couche de recouvrement et/ou un scellement.

10. Structure de couches selon la revendication 9, **caractérisée en ce que** celle-ci est un système de revêtement, la composition de polymère durcissable étant un revêtement de matière plastique.

11. Structure de couches selon la revendication 10, **caractérisée en ce que** celle-ci est un sol ou un revêtement de protection ou un système d'étanchéité à l'eau.

12. Procédé de revêtement ou de collage d'un substrat minéral poreux, comprenant les étapes suivantes :
(i) l'application de l'amorce selon l'une quelconque des revendications 4 à 7 sur le substrat minéral poreux, notamment en une quantité suffisante pour la fermeture des pores,
(ii) le durcissement de l'amorce appliquée à température ambiante, notamment au moins jusqu'à l'accessibilité,
(iii) l'application d'au moins une couche d'une composition de polymère durcissable sur l'amorce appliquée et au moins partiellement durcie.

13. Procédé selon la revendication 12, **caractérisé en ce que** l'amorce est appliquée et/ou durcie à une température inférieure à 20 °C.

14. Produit de réaction issu de la mise en réaction d'au moins une amine de la formule (I) avec au moins un composé de carbonyle de la formule (II) et de l'hydrogène,
NH₂-CH₂-A-CH₂-NH₂ (I) 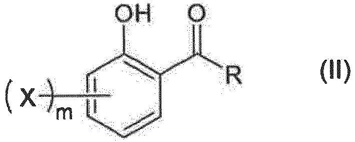
dans lesquelles
A représente un radical phénylène ou un radical cyclohexylène,
R représente un radical hydrogène ou un radical alkyle de 1 à 8 atomes C,
m représente 0 ou 1, et
X représente un radical hydroxyle ou un radical méthyle ou un radical méthoxy,
l'amine et le composé de carbonyle étant hydrogénés catalytiquement avec de l'hydrogène moléculaire en un rapport en moles dans la plage allant de 1/0,8 à 1/1,1 dans un solvant sans isolement de l'imine formée en tant que produit intermédiiare et sans élimination de l'eau libérée,
**caractérisé en ce que** le produit de réaction présente une teneur en amine de la formule (III) dans la plage allant de 30 à 80 % en poids
